# EUROPEAN PATENT APPLICATION

(11) **EP 3 000 801 A1**
(43) Date of publication of application: **30.03.2016**
(21) Application number: 14801585.2
(22) Date of filing: 20.05.2014
(51) Int. Cl.: C07C 7/10, C07C 7/20, B01D 11/04

(54) **METHOD FOR SEPARATING AROMATIC COMPOUNDS CONTAINED IN NAPHTHA**

(30) Priority: 20.05.2013 KR 20130056619; 15.11.2013 KR 20130139235; 25.04.2014 KR 20140050152; 25.04.2014 KR 20140050151
(71) Applicant: Lotte Chemical Corporation, Dongjak-gu Seoul 07071 (KR)
(72) Inventor: KIM, Jin Hyung, Daejeon 305-805 (KR); KIM, Wang Gyu, Daejeon 305-720 (KR); CHO, Joung Mo, Daejeon 305-720 (KR); SEO, Young Jeong, Daejeon 305-707 (KR)
(74) Representative: Tischner, Oliver
(86) International application number: PCT/KR2014/004492
(87) International publication number: WO 2014/189254

(57) **Abstract**

The present invention relates to a method for separating aromatic compounds contained in naphtha which includes an extraction step of contacting specific ionic liquids and naphtha and a step of separating the aromatic compounds from naphtha contacted with the ionic liquids.

## Description

### [TECHNICAL FIELD]

The present invention relates to a method for separating aromatic compounds contained in naphtha, and more particularly to an environmentally friendly and economical separation method that is capable of removing aromatic compounds from naphtha through a simple process with high efficiency while eliminating the need for multi-stage processes or various processing devices.

### [BACKGROUND ART]

In naphtha generated during oil refining, aliphatic compounds such as butane, pentane, cyclopentane, heptane, and octane as well as main aromatic compounds such as benzene, toluene, mixed xylene, and ethylbenzene are contained in an amount of about 4 % to 15 %.

If the main aromatic compounds contained in naphtha are selectively removed, the yield of ethylene and propylene is increased due to an increase in furnace cracking efficiency upon thermal cracking of naphtha and the amount of fuel used during the cracking decreases, and thereby a reduction in the amount of carbon dioxide by up to 10 % is achieved.

Specifically, in the case of removing about 90 % of the aromatic compounds from naphtha, it is known that the yield of ethylene and propylene increases by about 3 %.

In addition, there is an advantage of increasing the life of coils by reducing the occurrence of short-circuits in the naphtha cracker coil.

As a typical process for separating aromatic compounds contained in naphtha, the sulfolane process of UOP LLC is known.

The sulfolane process is a process of separating aromatic compounds from C6∼C8 Heart Cut (HTPG) derived from a hydrogenation process by using an extraction distillation method. Since the sulfolane has a higher boiling point than the C8 aromatic compound, it is possible to easily separate the aromatic compound from the extraction solvent. Therefore, the sulfolane is used as an extraction solvent.

However, such a sulfone process has problems in terms of energy, cost, and environmental aspects as described below.

First, in terms of energy, the sulfolane process involves a total of two distillation columns, and refers to a process which includes separating benzene from a first distillation column (extraction process), transferring the fraction having a high boiling point to a second distillation column (stripper) and separating toluene again through the distillation, wherein toluene is separated from the first distillation column at the maximum concentration and then remixed and transferred to the second distillation column, and thus toluene leads to the remixing phenomenon which is repeated in the first and second distillation columns. Thus, there is a problem that energy is inefficiently consumed.

Also, in terms of cost, because the sulfolane process is a large-scale process involving an extraction distillation column and a plurality of devices for recycling a stripper and an extraction solvent, the operation costs involved in the use of a distillation device for separation and a heat exchanger among various devices are high.

Further, in terms of the environmental aspects, sulfolane is a typical polar material. Sulfolane has advantages in that it is stable to heat and hydrolysis and has a high boiling point, but sulfolane also has disadvantages in that it has low selectivity for extracting aromatic compounds as well as aliphatic compounds and that a small amount of sulfolane is mixed with the aliphatic compounds, thus causing loss of the solvent.

Further, sulfolane starts to be decomposed at 200 °C. When exceeding 260 °C, it shows a high decomposition rate.

This means that the decomposition of the solvent occurs when a high temperature is applied for the regeneration of the solvent in the regeneration column.

Furthermore, the presence of oxygen in the process accelerates the decomposition of sulfolane. At this time, a generated acid gas results in corrosion in the process.

In addition to the sulforane process, some methods for separating aromatic compounds from naphtha are known.

As an example, U.S. Patent No. 5,849,981 relates to adsorptive separation, and more particularly discloses a method for selectively separating aromatic components using an adsorbent obtained by substituting one or more barium or potassium ions in zeolite.

However, in the case of using such a method, there are disadvantages in that the absorption capacity is limited, and a high temperature and high vacuum are required during desorption.

Furthermore, U.S. Patent No. 4,914,064 discloses a method for separation with a membrane, and more particularly, a method for separating aromatic components and aliphatic components using an anisotropic elastomeric polymer membrane prepared from a polyurea/urethane copolymer.

However, such a liquid membrane method has disadvantages in that the method for manufacturing the particular membrane used is difficult and the cost is expensive.

Furthermore, this method has disadvantages in that some membrane components are lost by an introduced gas and it is difficult to maintain separation efficiency for a long period of time, thus lowering efficiency.

In addition, a method for removing aromatic compounds from naphtha by double separation extraction using a halide-based ionic liquid as an extraction solvent of the liquid-liquid extraction method is known.

However, there is a high possibility that, when having F-, Cl-, Br-, or I- at the anion part of the ionic liquid, these are reacted with water used or partially contained during the process and thereby it is highly likely to be decomposed into hydrofluoric acid, hydrochloric acid, hydrobromic acid, hydrochloric acid, or hydroiodic acid, which cause toxicity and corrosion. Further, there is a problem that, due to relatively low electronegativity, the interaction with aromatic compounds is weak, thus decreasing the extraction efficiency of the main aromatic compounds.

### [Prior Art Document]

### [Patent Document]

(Patent Document 1) US Patent No. 5,849,981
(Patent Document 2) US Patent No. 4,914,064

### [DISCLOSURE OF INVENTION]

### [TECHNICAL PROBLEM]

It is an object of the present invention to provide an environmentally friendly and economical separation method that is capable of removing aromatic compounds from naphtha through a simple process with high efficiency while eliminating the need for multi-stage processes or various processing devices.

### [TECHNICAL SOLUTION TO PROBLEM]

In order to achieve these objects, and other objects which will become apparent from the description, the present invention provides a method for separating aromatic compounds contained in naphtha which includes the steps of: contacting naphtha with ionic liquids including one or more compounds selected from the group consisting of a triethylene glycol, a bis-imidazolium-based ionic solvent, a bis-piperidinium-based ionic solvent, a bis-pyrrolidinium-based ionic solvent, a bis-morpholinium-based ionic solvent, a bis-imidazolium-based salt including an ether group, a bis-piperidinium-based salt including an ether group, a bis-pyrrolidinium-based salt including an ether group, and a bis-morpholinium-based salt including an ether group; and separating aromatic compounds from the naphtha contacted with the ionic liquids.

Below, the method for separating aromatic compounds contained in naphtha in accordance with specific embodiments of the present invention will be described in more detail.

The term "alkylene" as used herein refers to a divalent functional group derived from an alkane.

According to one embodiment of the present invention, a method for separating aromatic compounds contained in naphtha which includes the steps of: contacting naphtha with ionic liquids including one or more compouds selected from the group consisting of a triethylene glycol, a bis-imidazolium-based ionic solvent, a bis-piperidinium-based ionic solvent, a bis-pyrrolidinium-based ionic solvent, a bis-morpholinium-based ionic solvent, a bis-imidazolium-based salt including an ether group, a bis-piperidinium-based salt including an ether group, a bis-pyrrolidinium-based salt including an ether group, and a bis-morpholinium-based salt including an ether group; and separating aromatic compounds from the naphtha contacted with the ionic liquids can be provided.

The present inventors found through numerous experiments that, when conducting the step of contacting naphtha with the ionic liquids including the above-described particular compounds and the step of separating aromatic compounds from the naphtha contacted with the ionic liquids, the aromatic compounds can be removed from naphtha through a simple process with high efficiency while eliminating the need for multi-stage processes or processing devices. The present invention has been completed on the basis of such a finding.

In the process of using the ionic liquids including the above-described particular compounds, components having toxicity or causing corrosion are not generated, thus allowing a more environmentally friendly process to be implemented.

Further, the aromatic compounds can be more effectively removed from naphtha with high efficiency even when conducting only the extraction step using the ionic liquids including the above-described particular compounds. Therefore, the need can be eliminated for complicated processes such as a multi-step extraction process or a purification process or installation of various devices, thus allowing a more economical process to be implemented.

The ionic liquid (IL) is a liquid composed of ions as defined in P. Wasserschied, T. Welton, Ionic Liquid in Synthesis, 2nd Ed, Wiley-VCH, 2008. In a broad sense, ionic liquid refers to a molten salt present as a liquid at a relatively low temperature of less than 100 °C.

Since the ionic liquid can be designed to have cations and anions according to its intended application, a range of application such as a catalyst, a reaction solvent, a separation medium, or an electrolyte solvent is broad.

In particular, the ionic liquid (IL) may be non-volatile and thermally stable as well as having high solubility and selectivity for metals, organic matter, and organic metals.

In the method for separating aromatic compounds contained in naphtha in accordance with the above embodiment of the invention, the ionic liquids including one or more compounds selected from the group consisting of a triethylene glycol, a bis-imidazolium-based ionic solvent, a bis-piperidinium-based ionic solvent, a bis-pyrrolidinium-based ionic solvent, a bis-morpholinium-based ionic solvent, a bis-imidazolium-based salt including an ether group, a bis-piperidinium-based salt including an ether group, a bis-pyrrolidinium-based salt including an ether group, and a bis-morpholinium-based salt including an ether group can be used.

Specifically, the bis-imidazolium-based salt including an ether group can include a cation in which two imidazolium-based rings are linked via a functional group including one or more ether groups having a total carbon number of 2 to 20, and a halide-based anion having hydrophobicity. The bis-piperidinium-based salt including an ether group can include a cation in which two piperidinium-based rings are linked via a functional group including one or more ether groups having a total carbon number of 2 to 20, and a halide-based anion having hydrophobicity. The bis-pyrrolidinium-based salt including an ether group can include a cation in which two pyrrolidinium-based rings are linked via a functional group including one or more ether groups having a total carbon number of 2 to 20, and a halide-based anion having hydrophobicity. The bis-morpholinium-based salt including an ether group can include a cation in which two morpholinium-based rings are linked via a functional group including one or more ether groups having a total carbon number of 2 to 20, and a halide-based anion having hydrophobicity.

The total carbon number of the ether group refers to the total number of carbon atoms except for the oxygen atom contained in the ether group.

Specific examples of the functional group including one or more ether groups having a total carbon atom number of 2 to 20 may include a functional group to which 1 to 10 ether groups having a total carbon number of 2 to 20 are linked.

The bis-imidazolium-based salt including an ether group, the bis-piperidinium-based salt including an ether group, the bis-pyrrolidinium-based salt including an ether group, and the bis-morpholinium-based salt including an ether group, which are included in the above ionic liquids, include two parts having a monovalent cation and two parts having an anion, respectively. The parts of interaction (hydrogen bond, π-interaction, etc.) with aromatic compounds contained in naphtha increase so the aromatic compounds contained in naphtha can be more efficiently extracted.

The bis-imidazolium-based salt including an ether group, the bis-piperidinium-based salt including an ether group, the bis-pyrrolidinium-based salt including an ether group, or the bis-morpholinium-based salt including an ether group are configured such that two imidazolium-based rings, piperidinium-based rings, pyrrolidinium-based rings, or morpholinium-based rings are linked via a functional group including one or more ether groups having a total carbon number of 2 to 20, wherein the imidazolium-based rings, the piperidinium-based rings, the pyrrolidinium-based rings, or the morpholinium-based rings have aromatic structures. Therefore, the above-described salts can easily form π-interaction with the aromatic compounds contained in naphtha, thus more increasing the extraction efficiency.

Further, since the bis-imidazolium-based salt including an ether group, the bis-piperidinium-based salt including an ether group, the bis-pyrrolidinium-based salt including an ether group, or the bis-morpholinium-based salt including an ether group have an ether group, these salts can easily form a hydrogen bond with the aromatic compounds contained in naphta, thus increasing the extraction efficiency.

Further, the bis-imidazolium-based salt including an ether group, the bis-piperidinium-based salt including an ether group, the bis-pyrrolidinium-based salt including an ether group, or the bis-morpholinium-based salt including an ether group may include an ether group and thereby include a halide-based anion having hydrophobicity, for example, (CF₃SO₂)₂N⁻, BF₄-, or PF₆⁻.

However, since such halide-based anions having hydrophobicity have greater electronegativity as compared with halogen ions (F-, Cl-, Br-, or I-) and two may be present in the above-described salt compounds, interactions such as hydrogen bonds with the aromatic compounds contained in naphtha can be maximized, thereby more efficiently extracting the aromatic compounds contained in naphtha.

Since the bis-imidazolium-based salt including an ether group, the bis-piperidinium-based salt including an ether group, the bis-pyrrolidinium-based salt including an ether group, or the bis-morpholinium-based salt including an ether group do not have substantial viscosity, the mass transfer of aromatic components (transfer from naphtha to ionic liquids) can be activated, thereby increasing the extraction efficiency of the aromatic components.

Also, since four kinds of the salt compounds as mentioned above have a very stable structure, the extracting performance of the aromatic compounds contained in naphtha can be maintained for a long period of time.

Further, the ionic liquids used in the separation method according to the above embodiment of the invention show hydrophobicity and so have almost no affinity to water. Therefore, unlike conventional ionic liquids including F-, Cl-, Br-, or I-based anions, since these ionic liquids do not react with water at all, harmful substances such as a hydrohalogenic acid (for example, hydrofluoronic acid) are not discharged.

Furthermore, since the ionic liquids used in the separation method according to the above embodiment of the invention have no own vapor pressure due to a boiling point (bp) of more than about 400 °C, there is no risk that the solvent is lost upon its re-use, in constrast with conventional organic solvent extraction agents. Further, these ionic liquids do not require a separate process so are cost-effective. In addition, components that cause environmental pollution are not discharged so it is possible to design an environmentally friendly process.

The bis-imidazolium-based salt including an ether group may include a compound represented by the following Formula 1.

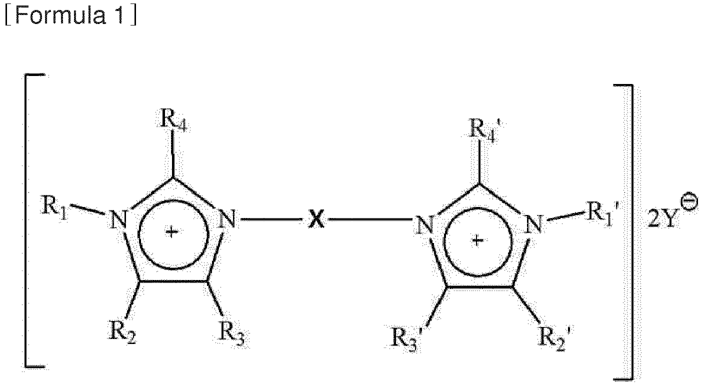

In the above Formula 1, R₁ and R₁' may be the same as or different from each other and are each independently a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms, and R₂, R₂', R₃, R₃', R₄, and R₄' may be the same as or different from each other and are each independently hydrogen, a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms.

Further, in the above Formula 1, Y is (CF₃SO₂)₂N⁻, BF₄-, or PF₆⁻, and X is the following Formula 1 a or Formula 1 b.

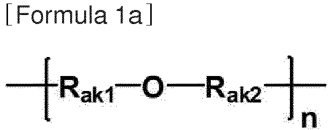

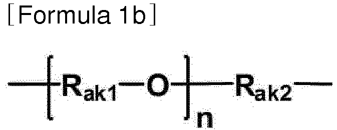

In the above Formula 1 a or Formula 1 b, Rₐₖ₁ and Rₐₖ₂ are each independently a linear or branched alkylene group having 1 to 10 carbon atoms, and n is an integer from 1 to 5.

The bis-piperidinium-based salt including an ether group may include a compound represented by the following Formula 2.

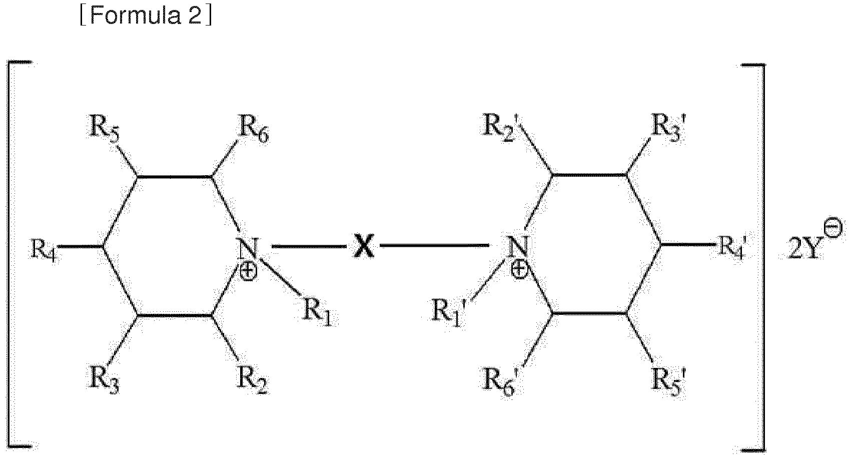

In the above Formula 2, R₁ and R₁' may be the same as or different from each other and are each independently a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms, and R₂, R₂', R₃, R₃', R₄, R₄', R₅, R₅', R₆, and R₆' may be the same as or different from each other and are each independently hydrogen, a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms.

Further, in the above Formula 2, Y is (CF₃SO₂)₂N⁻, BF₄-, or PF₆⁻, and X is the following Formula 2a or Formula 2b.

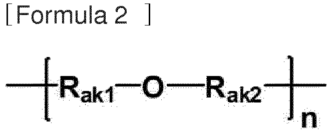

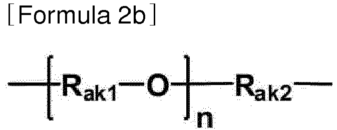

In the above Formula 2a or Formula 2b, Rₐₖ₁ and Rₐₖ₂ are each independently a linear or branched alkylene group having 1 to 10 carbon atoms, and n is an integer from 1 to 5.

The bis-pyrrolidinium-based salt including an ether group may include a compound represented by the following Formula 3.

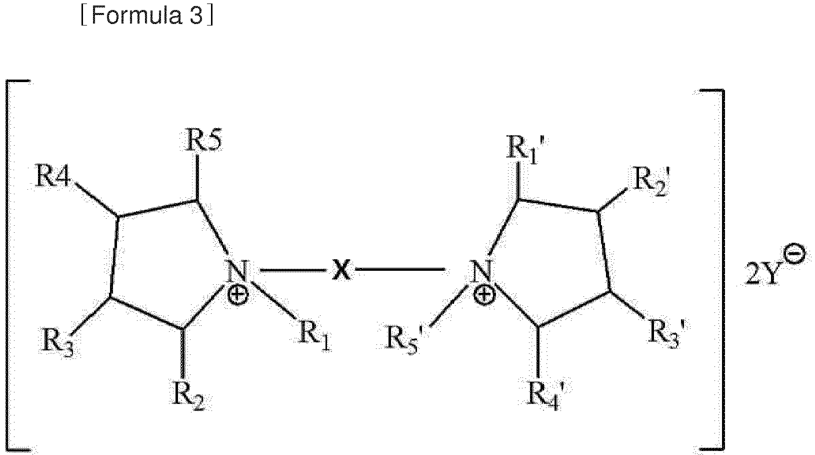

In the above Formula 3, R₁ and R₁' may be the same as or different from each other and are each independently a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms, and R₂, R₂', R₃, R₃', R₄, R₄', R₅, and R₅' may be the same as or different from each other and are each independently hydrogen, a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms.

Further, in the above Formula 3, Y is (CF₃SO₂)₂N⁻, BF₄-, or PF₆⁻, and X is the following Formula 3a or Formula 3b.

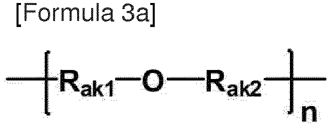

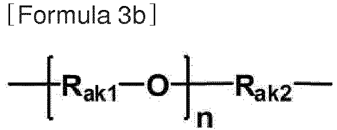

In the above Formula 3a or Formula 3b, Rₐₖ₁ and Rₐₖ₂ are each independently a linear or branched alkylene group having 1 to 10 carbon atoms, and n is an integer from 1 to 5.

The bis-morpholium-based salt including an ether group may include a compound represented by the following Formula 4.

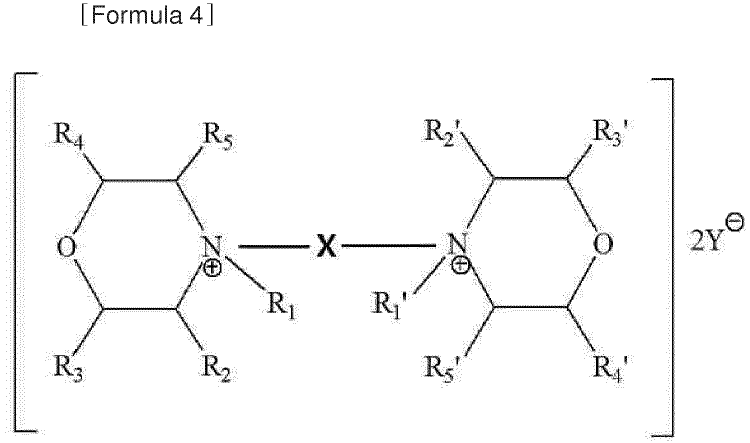

In the above Formula 4, R₁ and R₁' may be the same as or different from each other and are each independently a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms, and R₂, R₂', R₃, R₃', R₄, R₄', R₅, and R₅' may be the same as or different from each other and are each independently hydrogen, a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms.

Further, in the above Formula 4, Y is (CF₃SO₂)₂N⁻, BF₄-, or PF₆⁻, and X is the following Formula 4a or Formula 4b.

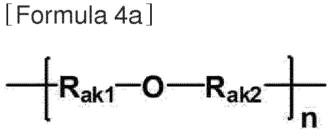

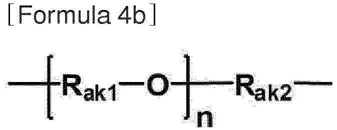

In the above Formula 4a or Formula 4b, Rₐₖ₁ and Rₐₖ₂ are each independently a linear or branched alkylene group having 1 to 10 carbon atoms, and n is an integer from 1 to 5.

Specific examples of the comounds contained in the above ionic liquids may include compounds represented by the following Formulas 11 to 29.

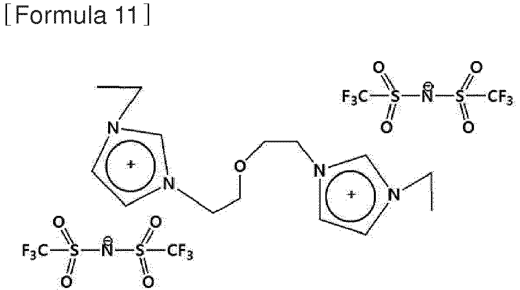

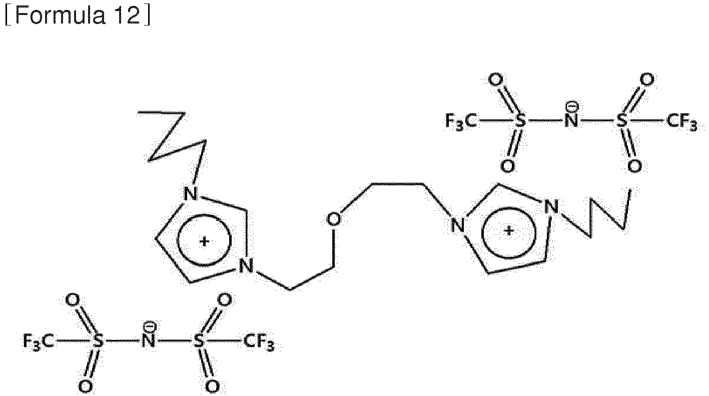

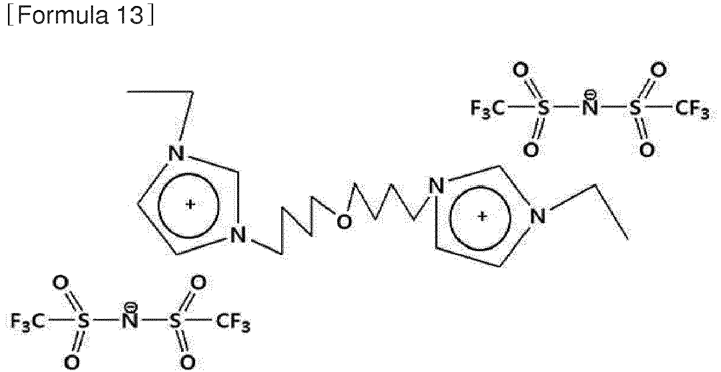

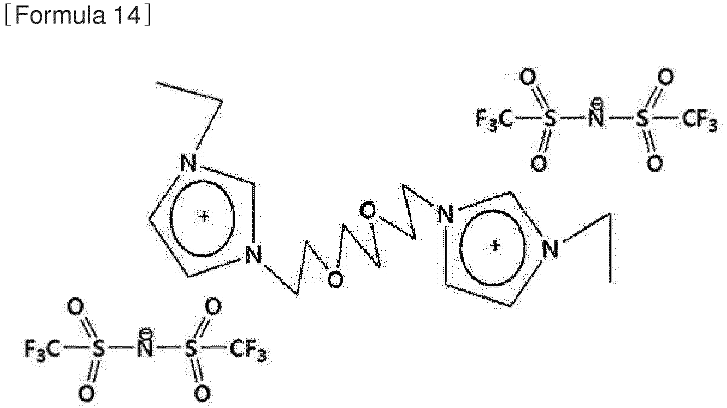

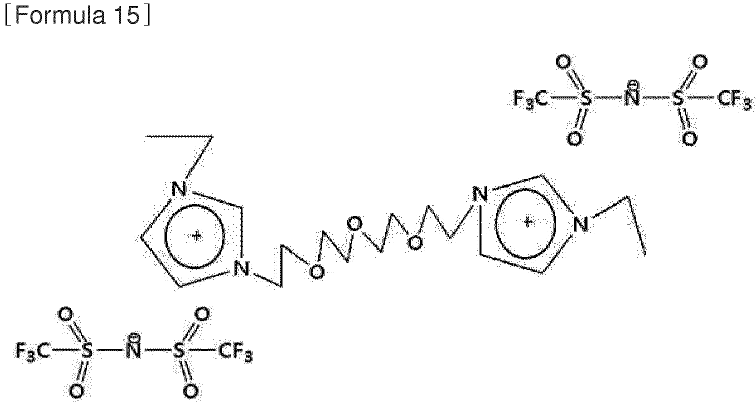

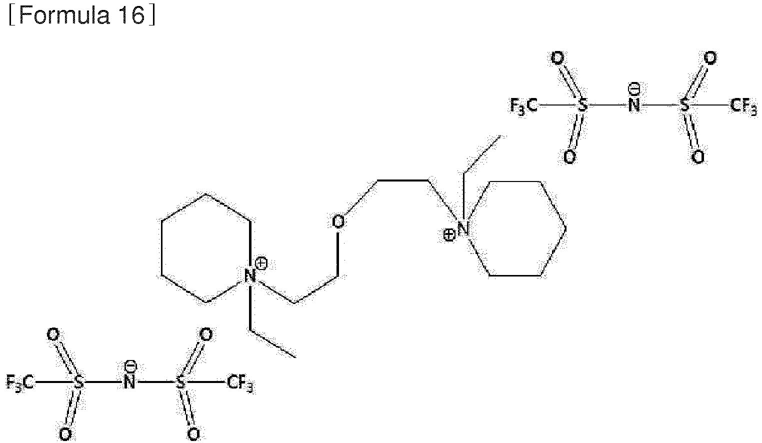

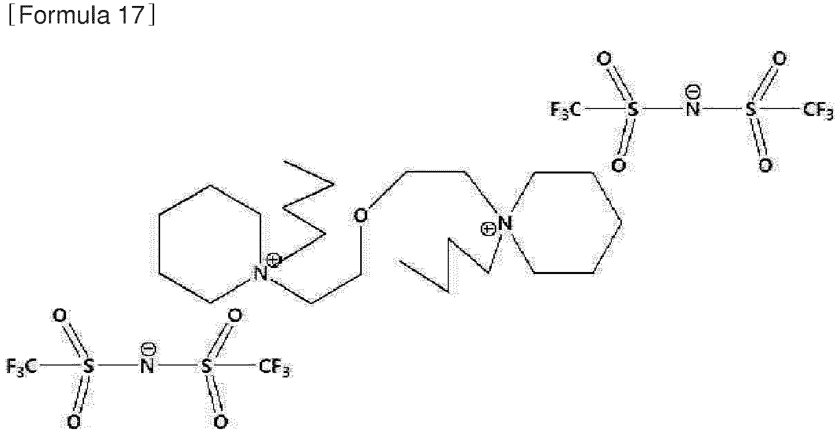

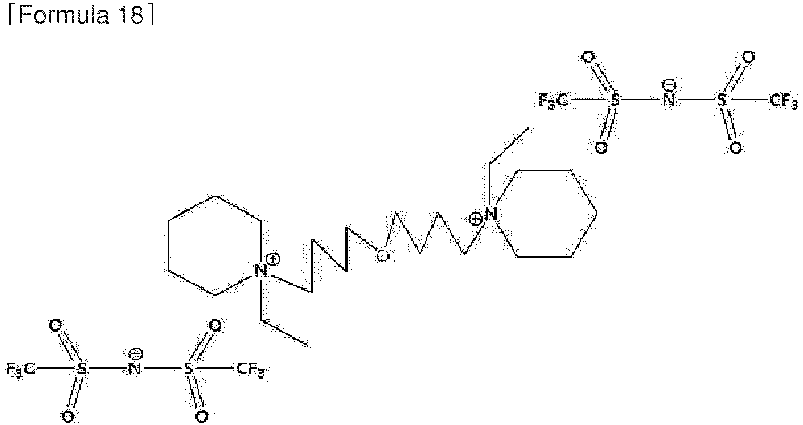

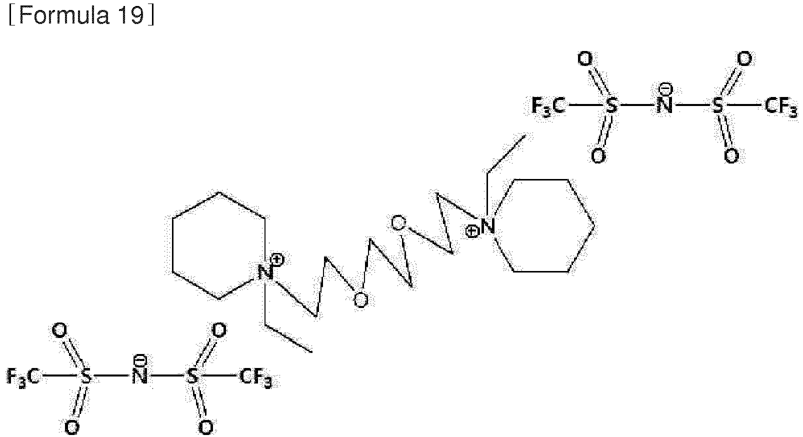

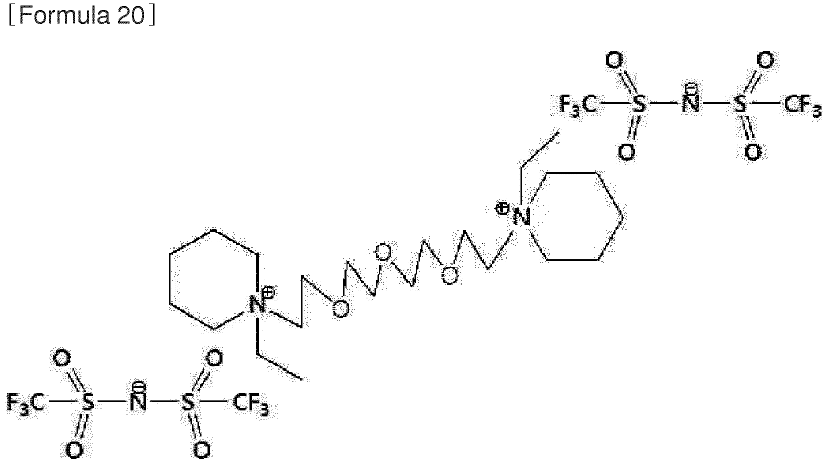

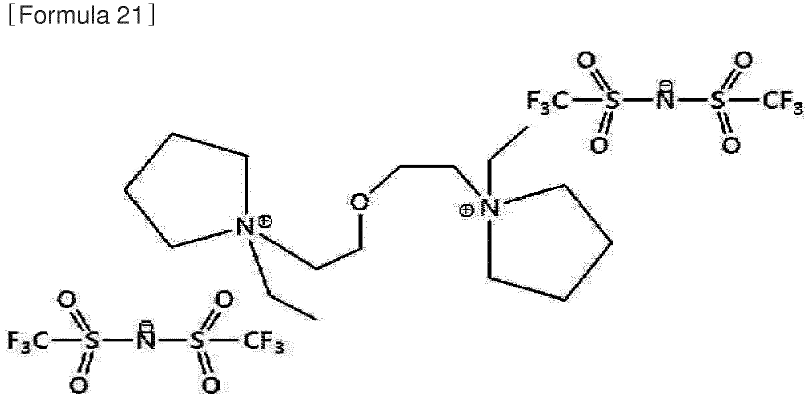

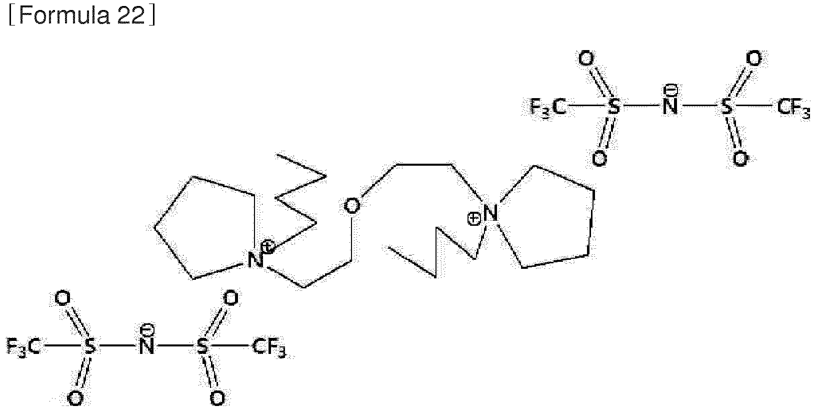

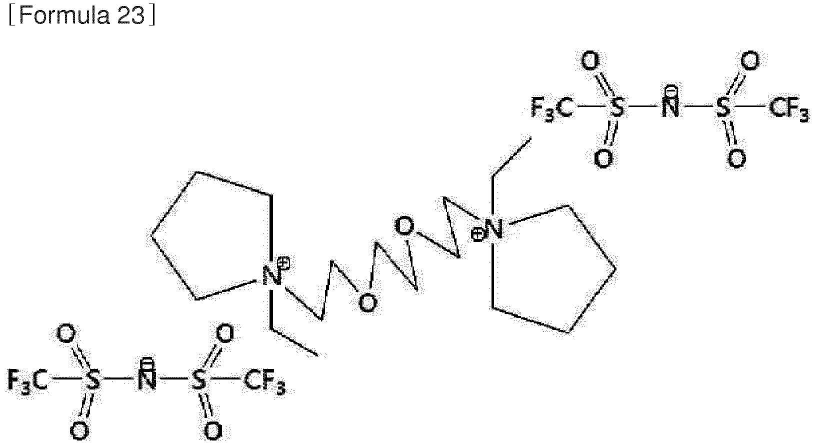

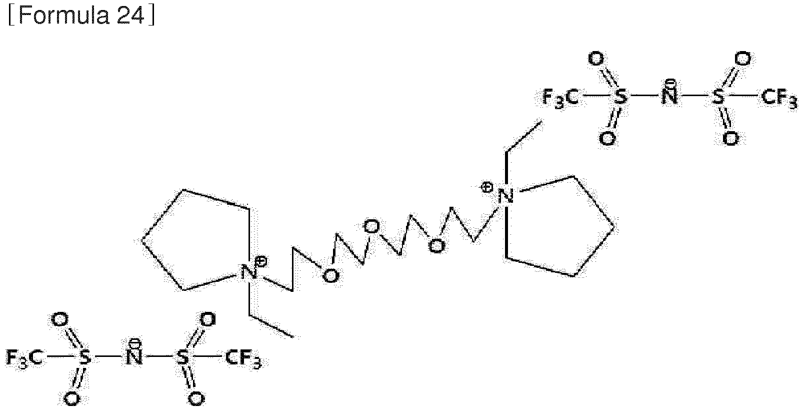

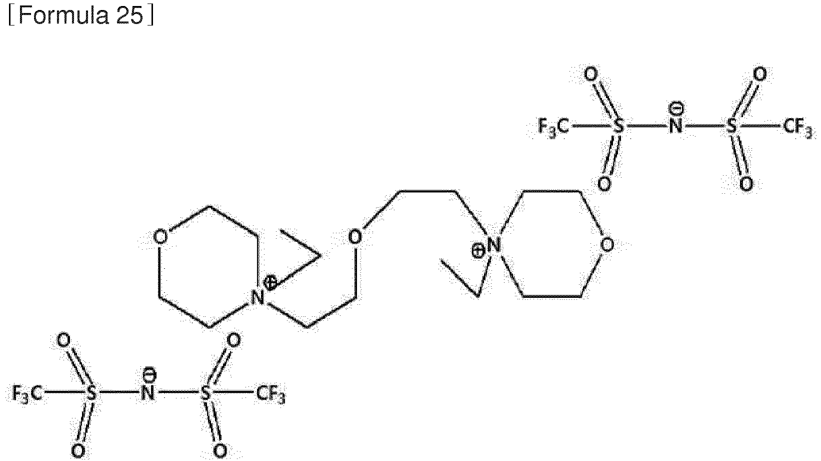

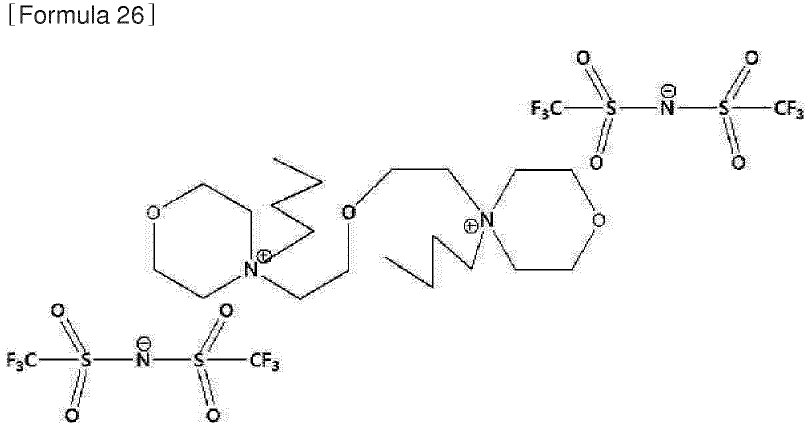

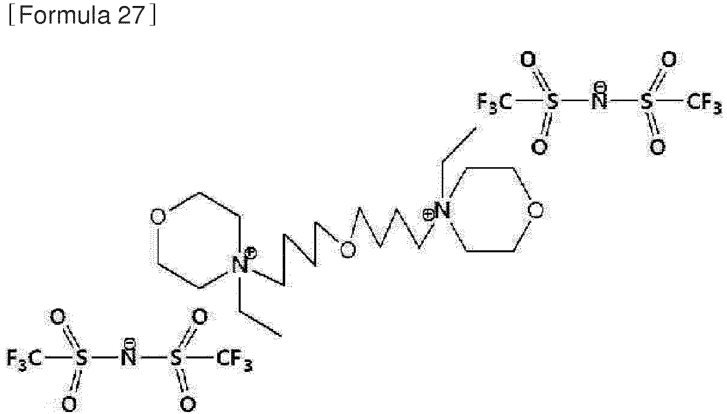

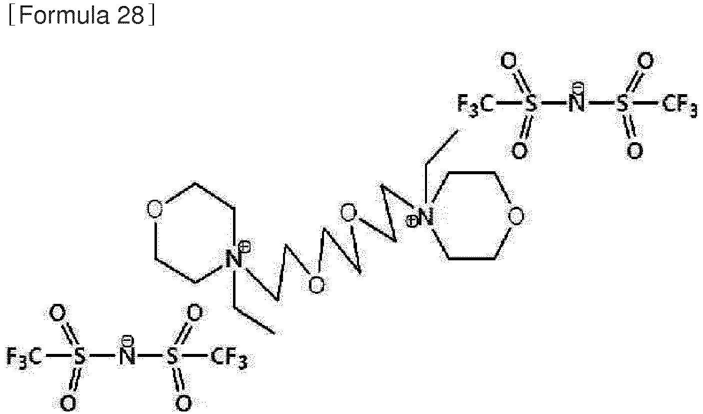

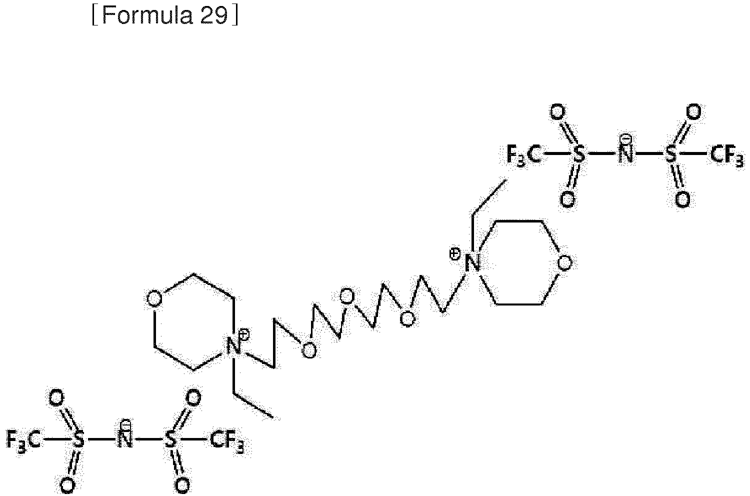

The bis-imidazolium-based salt including an ether group may include one or more compounds selected from the group consisting of [2-(1-ethylimidazolium-3-yl-ethoxy)-ethyl]-1-ethylimidazolium bis(trifluoromethanesulfonyl)imide, [2-(1-butylimidazolium-3-yl-ethoxy)-ethyl]-1-butylimidazolium bis(trifluoromethanesulfonyl)imide, [4-(1-ethylimidazolium-3-yl-butoxy)-butyl]-1-ethylimidazolium bis(trifluoromethanesulfonyl)imide, {2-[2-(1-ethylimidazolium-3-yl-ethoxy)-ethoxy]-ethyl}-1-ethylimidazolium bis(trifluoromethanesulfonyl) imide, (2-{2-[2-(1-ethylimidazolium-3-yl-ethoxy)-ethoxy]-ethoxy}-ethyl)-1-ethylimidazolium bis(trifluoromethanesulfonyl)imide, [2-(1-ethylimidazolium-3-yl-ethoxy)ethyl]-1-ethylimidazolium bis(tetrafluoroborate), [2-(1-butylimidazolium-3-yl-ethoxy)ethyl]-1-butylimidazolium bis(tetrafluoroborate), [4-(1-ethylimidazolium-3-yl-butoxy)-butyl]-1-ethylimidazolium bis(tetrafluoroborate), {2-[2-(1-ethylimidazolium-3-yl-ethoxy)-ethoxy]-ethyl}-1-ethylimidazolium bis(tetrafluoroborate), (2-{2-[2-(1-ehtylimidazolium-3-yl-ethoxy)-ethoxy]-ethoxy}-ethyl)-1-ethylimidazolium bis(tetrafluoroborate), [2-(1-ethylimidazolium-3-yl-ethoxy)ethyl]-1-ethylimidazolium bis(hexafluorophosphate), [2-(1-butylimidazolium-3-yl-ethoxy) ethyl]-1-butylimidazolium bis(hexafluorophosphate), [4-(1-ethylimidazolium-3-yl-butoxy)-butyl]-1-ethylimidazolium bis(hexafluorophosphate), {2-[2-(1-ethylimidazolium-3-yl-ethoxy)-ethoxy]-ethyl}-1-ethylimidazolium bis(hexafluorophosphate), and (2-{2-[2-(1-ethylimidazolium-3-yl-ethoxy)-ethoxy]-ethoxy}-ethyl)-1-ethylimidazolium bis(hexafluorophosphate).

The bis-piperidinium-based salt including an ether group may include one or more compounds selected from the group consisting of [2-(1-ethylpiperidinium-1-yl-ethoxy)-ethyl]-1-ethylpiperidinium bis(trifluoromethanesulfonyl)imide, [2-(1-butylpiperidinium-1-yl-ethoxy)-ethyl]-1-butylpiperidinium bis(trifluoromethanesulfonyl)imide, [4-(1-ethylpiperidinium-1-yl-butoxy)-butyl]-1-ethyl piperidinium bis(trifluoromethane sulfonyl)imide, {2-[2-(1-ethylpiperidinium-1-yl-ethoxy)-ethoxy]-ethyl}-1-ethylpiperidinium bis(trifluoromethane sulfonyl)imide, (2-{2-[2-(1-ethylpiperidinium-1-yl-ethoxy)-ethoxy]-ethoxy}-ethyl)-1-ethylpiperidinium bis(trifluoro methanesulfonyl)imide, [2-(1-ethylpiperidinium-1-yl-ethoxy)-ethyl]-1-ethylpiperidinium bis(tetrafluoroborate), [2-(1-butylpiperidinium-1-yl-ethoxy)-ethyl]-1-butylpiperidinium bis(tetrafluoroborate), [4-(1-ethylpiperidinium-1-yl-butoxy)-butyl]-1-ethylpiperidinium bis(tetrafluoroborate), {2-[2-(1-ethylpiperidinium-1-yl-ethoxy)-ethoxy]-ethyl}-1-ethylpiperidinium bis(tetrafluoroborate), (2-{2-[2-(1-ethylpiperidinium-1-yl-ethoxy)-ethoxy]-ethoxy}-ethyl)-1-ethylpiperidinium bis(tetrafluoroborate), [2-(1-ethylpiperidinium-1-yl-ethoxy)ethyl]-1-ethylpiperidinium bis(hexafluorophosphate), [2-(1-butylpiperidinium-1-yl-ethoxy) ethyl]-1-butylpiperidinium bis(hexafluorophosphate), [4-(1-ethylpiperidinium-1-yl-butoxy)-butyl]-1-ethylpiperidinium bis(hexafluorophosphate), {2-[2-(1-ethylpiperidinium-1-yl-ethoxy)-ethoxy]-ethyl}-1-ethylpiperidinium bis(hexafluorophosphate), and (2-{2-[2-(1-ethylpiperidinium-1-yl-ethoxy)-ethoxy]-ethoxy}-ethyl)-1-ethylpiperidium bis(hexafluorophosphate).

The bis-pyrrolidinium-based salt including an ether group may include one or more compounds selected from the group consisting of [2-(1-ethylpyrrolidinium-1-yl-ethoxy)ethyl]-1-ethylpyrrolidinium bis(trifluoromethanesulfonyl)imide, [2-(1-butylpyrrolidinium-1-yl-ethoxy)-ethyl]-1-butylpyrrolidinium bis(trifluoromethanesulfonyl)imide, [4-(1-ethylpyrrolidinium-1-yl-butoxy)-butyl]-1-ethyl]-1-ethylpyrrolidinium bis(trifluoromethanesulfonyl)imide, {2-[2-(1-ethylpyrrolidinium-1-yl-ethoxy-ethoxy]-ethyl}-1-ethylpyrrolidinium bis(trifluoromethanesulfonyl)imide, (2-{2-[2-(1-ethylpyrrolidinium-1-yl-ethoxy)-ethoxy]-ethoxy}-ethyl)-1-ethylpyrrolidinium bis(trifluoromethanesulfonyl)imide, [2-(1-ethylpyridinium-1-yl-ethoxy)-ethyl]-1-ethylpyrrolidinium bis(tetrafluoroborate), [2-(1-butylpyrrolidinium-1-yl-ethoxy)-ethyl]-1-butylpyrrolidinium bis(tetrafluoroborate), [4-(1-ethylpyrrolidinium-1-yl-butoxy)-butyl]-1-ethylpyrrolidinium bis(tetrafluoroborate), {2-[2-(1-ethylpyrrolidinium-1-ethoxy)-ethoxy]-ethyl}-1-ethyl-pyrrolidinium bis(tetrafluoroborate), (2-{2-[2-(1-ethylpyrrolidinium-1-yl-ethoxy)-ethoxy]-ethoxy}-ethyl)-1-ethylpyrrolidinium bis(tetrafluoroborate), [2-(1-ethylpyrrolidinium-1-yl-ethoxy)-ethyl]-1-ethylpyrrolidinium bis(hexafluorophosphate), [2-(1-butylpyrrolidinium-1-yl-ethoxy)-ethyl]-1-butylpyrrolidinium bis(hexafluorophosphate), [4-(1-ethylpyrrolidinium-1-yl-butoxy)-butyl]-1-ethylpyrrolidinium-bis(hexafluorophosphate), {2-[2-(1-ethylpyrrolidinium-1-yl-ethoxy)-ethoxy]-ethyl}-1-ethylpyrrolidinium bis(hexafluorophosphate), and (2-{2-[2-(1-ethylpyrrolidinium-1-yl-ethoxy)-ethoxy]-ethoxy}-ethyl)-1-ethylpyrrolidinium.

The bis-morpholinium-based salt including an ether group may include one or more compounds selected from the group consisting of [2-(4-ethylmorpholinium-4-yl-ethoxy)-ethyl]-4-ethylmorpholinium bis(trifluoromethane sulfonyl)imide, [2-(4-butymorpholinium-4-yl-ethoxy)-ethyl]-4-butylmorpholinium bis(trifluoromethanesulfonyl)imide, [4-(4-ethylmorpholinium-4-yl-butoxy)-butyl]-4-ethylmorpholinium bis(trifluoromethanesulfonyl)imide, {2-[2-(4-ethylpyrrolidinium-4-yl-ethoxy)-ethoxy]-ethyl)-4-ethylmorpholinium bis(trifluoromethane sulfonyl)imide, (2-{2-[2-(4-ethylmorpholinium-4-yl-ethoxy)-ethoxy}-ethyl)-4-ethylmorpholinium bis(trifluoro methane sulfonyl)imide, [2-(4-ethylmorpholinium-4-yl-ethoxy)-ethyl]-4-ethylmorpholinium bis(tetrafluoroborate), [2-(4-butylmorpholinium-4-yl-ethoxy)-ethyl]-4-butylmorpholinium bis(tetrafluoroborate), [4-(4-ethylmorpholinium-4-yl-butoxy)-butyl]-4-ethylmorpholinium bis(tetrafluoroborate), {2-[2-(4-ethylmorpholinium-4-yl-ethoxy)-ethoxy]-ethyl}-4-ethylmorpholinium(tetrafluoroborate), (2-{2-[2-(4-ethylmorpholinium-4-yl-ethoxy)-ethoxy]-ethoxy}-ethyl)-4-ethylmorpholinium bis(tetrafluoroborate), [2-(4-ethylmorpholinium-4-yl-ethoxy)-ethyl]-4-ethylmorpholinium bis(hexafluorophosphate), [2-(4-butylmorpholinium-4-yl-ethoxy)-ethyl]-4-butylmorpholinium bis(hexafluorophosphate), [4-(4-ethylmorpholinium-4-yl-butoxy)-butyl]-4-ethylmorpholinium bis(hexafluorophosphate), {2-[2-(4-ethylmorpholinium-4-yl-ethoxy)-ethoxy]-ethyl}-4-ethylmorpholinium (hexafluorophosphate), and (2-{2-[2-(4-ethylmorpholinium-4-yl-ethoxy)-ethoxy]-ethoxy}-ethyl)-4-ethylmorpholinium bis(hexafluorophosphate).

On the other hand, the bis-imidazolium-based ionic solvent that may be included in the ionic liquids may include one or more selected from the group consisting of 1,1'-(1,2-ethanediyl)bis(3-methylimidazolium) bis(trifluoromethanesulfonyl)imide, 1,1'-(1,4-butanediyl)bis(3-ethylimidazolium) bis(trifluoromethanesulfonyl)imide, 1,1'-(2,4'-butenediyl)bis(3-butylimidazolium) bis(trifluoromethanesulfonyl)imide, 1,1'-(1,4-butanediyl-2-one)bis(3-ethylimidazolium) bis(trifluoromethanesulfonyl)imide, 1,1'-(1,4-butanediyl-2,3-dione)bis(3-butylimidazolium) bis(trifluoromethanesulfonyl)imide, 1,1'-(1,4-butanediyl-2-ol)bis(3-methylimidazolium) bis(trifluoromethanesulfonyl) imide, 1,1'-(1,4-butanediyl-2,3-diol)bis(3-ethylimidazolium) bis(trifluoromethanesulfonyl)imide, 1,1'-(1,2-ethanediyl)bis(3-methylimidazolium) bis(tetrafluoroborate), 1,1'-(1,4-butanediyl) bis(3-ethylimidazolium) bis(tetrafluoroborate), 1,1'-(2,4-betenediyl)bis(3-butylimidazolium) bis(tetrafluoroborate), 1,1'-(1,4-butanediyl-2-one)bis(3-ethylimidazolium) bis(tetrafluoroborate), 1,1'-(1,4-butanediyl-2,3-dione)bis(3-ethyl imidazolium) bis(tetrafluoroborate), 1,1'-(1,4-butanediyl-2-ol) bis(3-ethylimidazolium) bis(tetrafluoroborate), 1,1'-(1,4-butanediyl-2,3-diol) bis(3-ethylimidazolium) bis(tetrafluoroborate), 1,1'-(1,2-ethanediyl)bis(3-methylimidazolium) bis(hexafluorophosphate), 1,1'-(1,4-butanediyl)bis(3-ethylimidazolium) bis(hexafluorophosphate), 1,1'-(2,4-butenediyl)bis(3-butylimidazolium) bis(hexafluorophosphate), 1,1'-(1,4-butanediyl-2-one) bis(3-ethylimidazolium) bis(hexafluorophosphate), 1,1'-(1,4-butanediyl-2,3-dione) bis(3-butylimidazolium) bis(hexafluorophosphate), 1,1'-(1,4-butanediyl-2-ol)bis(3-ethylimidazolium) bis(hexafluorophosphate), and 1,1'-(1,4-butanediyl-2,3-diol) bis(3-ethylimidazolium) bis(hexafluorophosphate).

Further, the bis-pyrrolidinium-based ionic solvent that may be included in the ionic liquids may include one or more selected from the group consisting of 1,1'-(1,4-butanediyl)bis(1-ethylpyrrolidinium) bis(trifluormethane sulfonyl)imide, 1,1'-(1,4-butanediyl)bis(1-butylpyrrolidinium)bis(trifluoromethanesulfonyl)imide, 1,1'-(1,4-butanediyl-2-ol)bis(1-ethylpyrrolidinium) bis(trifluoromethanesulfonyl)imide, 1,1'-(1,4-butanediyl-2,3-diol) bis(1-ethylpyrrolidinium) bis(trifluoromethane sulfonyl)imide, 1,1'-(2,3-butendiyl)bis(1-ethylpyrrolidinium) bis(trifluoromethanesulfonyl)imide, 1,1'-(1,4-butanediyl-2,3-dione) bis(1-ethylpyrrolidinium) bis(trifluoromethane sulfonyl)imide, 1,1'-(1,4-butanediyl) bis(1-ethylpyrrolidinium) bis(tetrafluoroborate), 1,1'-(1,4-butanediyl)bis(1-butylpyrrolidinium) bis(tetrafluoroborate), 1,1'-(1,4-butanediyl-2-ol)bis(1-ethylpyrrolidinium)bis(tetrafluoroborate), 1,1'-(1,4-butanediyl-2,3-diol)bis(1-ethylpyrrolidinium) bis(tetrafluoroborate), 1,1'-(2,3-butenediyl)bis(1-ethylpyrrolidinium) bis(tetrafluoroborate), 1,1'-(1,4-butanediyl-2,3-dione)bis(1-ethylpyrrolidinium) bis(tetrafluoroborate), 1,1'-(1,4-butanediyl)bis(1-ethylpyrrolidinium) bis(hexafluorophosphate), 1,1'-(4-butanediyl) bis(1-butylpyrrolidinium) bis(hexafluorophosphate), 1,1'-(1,4-butanediyl-2-ol) bis(1-ethylpyrrolidinium)bis(hexafluorophosphate), 1,1'-(1,4-butanediyl-2,3-diol) bis(1-ethylpyrrolidinium) bis(hexafluorophosphate), 1,1'-(2,3-butenediyl) bis(1-ethylpyrrolidinium) bis(hexafluorophosphate), and 1,1'-(1,4-butanediyl-2,3-dione) bis(1-ethylpyrrolidinium) bis(hexafluorophosphate).

Further, the bis-morpholinium-based ionic solvent may include one or more selected from the group consisting of 1,1'-(1,4-butanediyl)bis(1-ethylmorpholinium) bis(trifluoromethanesulfonyl)imide, 1,1'-(1,4-butanediyl) bis(1-butylmorpholinium) bis(trifluoro methane sulfonyl)imide, 1,1'-(1,4-butanediyl-2-ol) bis(1-ethylmorpholinium) bis(trifluoromethane sulfonyl)imide, 1,1'-(1,4-butanediyl-2,3-diol)bis(1-ethylmorpholinium) bis(trifluoromethane sulfonyl)imide, 1,1"-(2,3-butenediyl) bis(1-ethylmorpholinium) bis(trifluoromethanesulfonyl)imide, 1,1'-(1,4-butanediyl-2,3-dione) bis(1-ethylmorpholinium) bis(trifluoromethanesulfonyl)imide, 1,1'-(1,4-butanediyl)bis(1-ethylmorpholinium) bis(tetrafluoroborate), 1,1'-(1,4-butanediyl)bis(1-butylmorpholinium) bis(tetrafluoroborate), 1,1'-(1,4-butanediyl-2-ol) bis(1-ethylmorpholinium) bis(tetrafluoroborate), 1,1'-(1,4-butanediyl-2,3-diol) bis(1-ethylmorpholinium) bis(tetrafluoroborate), 1,1'-(2,3-butenediyl) bis(1-ethylmorpholinium) bis(tetrafluoroborate), 1,1'-(1,4-butanediyl-2,3-dione)bis(1-ethylmorpholinium)bis(tetrafluoroborate), 1,1'-(1,4-butanediyl) bis(1-ethylmorpholinium) bis(hexafluorophosphate), 1,1'-(1,4-butanediyl) bis(1-butylmorpholinium) bis(hexafluorophosphate), 1,1'-(1,4-butanediyl-2-ol) bis(1-ethyl-morpholinium) bis(hexafluorophosphate), 1,1'-(1,4-butanediyl-2,3-diol) bis(1-ethyl-morpholinium) bis(hexafluorophosphate), 1,1'-(2,3-butendiyl) bis(1-ethyl-morpholinium) bis(hexafluorophosphate), and 1,1'-(1,4-butanediyl-2,3-dione) bis(1-ethylmorpholinium)bis(hexafluorophosphate).

Further, the bis-piperidinium-based ionic solvent may include one or more selected from the group consisting of 1,1'-(1,4-butanediyl) bis(1-ethylpiperidinium) bis(trifluoromethane sulfonyl)imide, 1,1'-(1,4-butanediyl) bis(1-butylpiperidinium) bis(trifluoromethanesulfonyl) imide, 1,1'-(1,4-butanediyl-2-ol)bis(1-ethylpiperidinium) bis(trifluoromethanesulfonyl)imide, 1,1'-(1,4-butanediyl-2,3-diol)bis(1-ethylpiperidinium) bis(trifluoromethanesulfonyl)imide, 1,1' -(2,3-butanediyl)bis(1-ethylpiperidinium) bis(trifluoromethanesulfonyl)imide, 1,1'-(1,4-butanediyl-2,3-dione)bis(1-ethylpiperidinium) bis(trifluoromethanesulfonyl)imide, 1,1'-(1,4-butanediyl)bis(1-ethylpiperidinium)bis(tetrafluoroborate), 1,1'-(1,4-butandiyl)bis(1-butylpiperidinium) bis(tetrafluoroborate), 1,1'-(1,4-butanediyl-2-ol)bis(1-ethylpiperidinium) bis(tetrafluoroborate), 1,1'-(1,4-butanediyl-2,3-diol) bis(1-ethylpiperidinium) bis(tetrafluoroborate), 1,1'-(2,3-butenediyl)bis(1-ethylpiperidinium) bis(tetrafluoroborate), 1,1'-(1,4-butanediyl-2,3-dione) bis(1-ethylpiperidinium) bis(tetrafluoroborate), 1,1'-(1,4-butanediyl)bis(1-ethylpiperidinium) bis(hexafluorophosphate), 1,1'-(1,4-butanediyl) bis(1-butylpiperidinium) bis(hexafluorophosphate), 1,1'-(1,4-butanediyl-2-ol) bis(1-ethylpiperidinium) bis(hexafluorophosphate), 1,1'-(1,4-butanediyl-2,3-diol) bis(1-ethylpiperidinium) bis(hexafluorophosphate), 1,1'-(2,3-butenediyl) bis(1-ethylpiperidinium) bis(hexafluorophosphate), and 1,1'-(1,4-butanediyl-2,3-dione)bis(1-ethylpiperidinium) bis(hexafluorophosphate).

On the other hand, for naphtha, as a subject from which to extract and separate the aromatic compounds, conventional naphtha known in the art can be used without limitation.

For example, the naphtha may contain 4% to 15 % by weight of aromatic compounds, and in particular it may contain 4 % to 15 % by weight of aromatic compounds such as benzene, toluene, mixed xylene, and ethylbenzene.

As an example of the naphtha, aliphatic hydrocarbon compounds, for example, aliphatic hydrocarbon compounds including compounds such as butane (about 2.3 wt%), pentane (about 30.1 wt%), hexane (about 31.8 wt%), heptane (about 8.7 wt%), octane (about 8.1 wt%), nonane (about 2.0 wt%) and/or decane (about 7.0 wt%), and aromatic compounds, for example, aromatic compounds including compounds such as benzene (about 1.8 wt%), toluene (about 3.0 wt%), mixed xylene (about 3.2 wt%), and/or ethyl benzene (about 2.0 wt%) can be included.

On the other hand, in the method for separating aromatic compounds contained in naphtha in accordance with the above embodiment of the invention, the naphtha can be used in an amount of 0.05 % to 5 % by volume, or 0.1 % to 3 % by volume compared to the ionic liquids.

When the naphtha is used in an amount of less than 0.05 % by volume compared to the ionic liquids, the efficiency or productivity and economy of the method for separation of aromatic compounds contained in naphtha in accordance with the above one embodiment of the invention cannot be sufficiently secured, and for the recovery of aromatic compounds, it may be necessary to add additional process devices or process steps to the separation method in accordance with the above one embodiment.

If the used amount of the naphtha exceeds 5 % by volume compared to the ionic liquids, the extraction efficiency of the aromatic compounds can be lowered. In order to sufficiently separate the aromatic compounds from naphtha, it may be necessary to add the multi-step processes or additional process devices.

The step of contacting the ionic liquids and naphtha may be conducted at a temperature of 20 °Cto 100 °Cor a temperature of 25 °Cto 80 °C

If the step of contacting the ionic liquids and naphtha is conducted at a temperature of less than 20 °C, there are problems in that the viscosity of the ionic liquids is increased, the dispersion is lowered, and the mass transfer performance is decreased.

Further, if the step of contacting the ionic liquids and naphtha is conducted at a temperature of greater than 100 °C there may be a problem in that naphtha is vaporized and contact with an ionic liquid is not smoothly made so the extraction performance is reduced. In addition, due to energy consumption, production costs may be increased.

The time required for conducting the step of contacting the ionic liquid and naphtha is not particularly limited, but the contacting can be conducted for more than 1 min or more than 30 min so that the aromatic compounds can be in sufficient contact with the ionic liquids. Specifically, the contact can be conducted for 1 min to 20 h or for 30 min to 10 h.

The steps for separating the aromatic compounds from naphtha contacted with the ionic liquids can be conducted at least once, for example once to four times.

The steps for separating the aromatic compounds from naphtha contacted with the ionic liquids can include a step for separation by a liquid-liquid extraction method.

The liquid-liquid extraction method can use conventionally known methods and devices. It is possible to select an organic solvent that is used in consideration of the polarity and the like of the substance.

The method for separation of aromatic compounds contained in naphtha according to the above embodiment of the invention may further include a step for separating the raffinate obtained in the extraction step.

The raffinate refers to a component that is not extracted or adsorbed by the solvent extraction or molecular sieve extraction. In the extraction step, the raffinate refers to a residue in which aromatic compound components are separated from naphtha.

The method for separation of aromatic compounds contained in naphtha according to the above embodiment of the invention may further include a deaeration step for separating aromatic compounds from the ionic liquids.

The raffinate and the ionic liquids are introduced into the evaporator and then the deaeration step can be conducted.

The deaeration refers to an operation of removing gas dissolved in the liquid. In the present specification, the deaeration refers to vaporizing the aromatic compounds present in the ionic liquid and removing them.

The deaeration can be conducted at a temperature of 20 °C to 150 °C or 50 °C to 120 °C.

If the temperature during said deaeration is too low, the deaeration performance is lowered so a problem that the extraction efficiency is decreased when reusing the ionic liquids may occur.

Further, if the temperature during said deaeration is too high, a problem that variations in the ionic liquid and energy consumption increase may occur.

The deaeration can be conducted under the pressure of 1 mmHg to 200 mmHg or 50 mmHg to 100 mmHg.

If the pressure during said deaeration is too low, a problem that energy consumption increases may occur.

Furthermore, if the pressure during said deaeration is too high, a problem that deaeration performance is lowered and the extraction efficiency is lowered when reusing the inonic liquids may occur.

That is, the step of separating aromatic compounds from naphtha contacted with the ionic liquids may further include a step of conducting the deaeration at a temperature of 20 to 150 °C and a pressure of 1 to 200 mmHg.

The time required for conducting the deaeration is not particularly limited, but in order to sufficiently ensure the deaeration efficiency, the deaeration can be conducted for 5 min or more, specifically for 10 min to 300 min, or 30 min to 120 min.

The step of separating an aromatic compound from naphtha contacted with the ionic liquids may further include a step of contacting the ioinic liquid after the separation step with naphtha and reusing it.

For example, after the deaeration step, the raffinate and the aromatic compounds separated from the ionic liquids are discharged to a passage of the upper end of the evaporator, and the ionic liquid in which the aromatic compound has been separated is discharged to a passage of the lower end of the evaporator and again introduced into an ionic liquid inlet passage of the extraction column and reused.

In other words, the ionic liquids in which the aromatic compounds have been separated through an deaeration step can be reused without loss.

In the method for separating aromatic compounds contained in naphtha in accordance with the above one embodiment of the invention, the extraction step of contacting the ionic liquids and naphtha can be conducted in a pulsed extraction column.

The pulsed extraction column refers to an extraction column that imparts a pulsation to the continuation phase of the liquid-liquid countercurrent extraction column. By imparting a pulsation to the liquid, the pulsed extraction column utilizes a phenomenon in which a phase having a small volume ratio among two liquid phases is dispersed in the form of a drop within the other continuation phase.

Since the separation method of the above one embodiment of the invention utilizes a pulsed extraction column, the dispersibility increases and a mass transfer performance is remarkably improved, thereby increasing the efficiency for extracting aromatic compounds from naphtha.

In the pulsed extraction column, the pulse generating motor at the upper end is operated at a velocity of 10 m/s to 60 m/s or 20 m/s to 50 m/s, so a double tube pulsation-type column conducts a vertical reciprocating motion.

If the velocity of the pulsation generating motor is too slow and is less than 10 m/s, problems in that the vertical reciprocating motion of the pulsed extraction column is decreased and thus the dispersion and the mass transfer performance are reduced may occur, thus lowering the extraction efficiency of the aromatic compounds.

Further, if the velocity of the pulsation generating motor is too fast and is greater than 60 m/s, problems in that the functionability is lowered due to the abrasion of the device and production costs cab increase due to consumption of energy may occur.

If the extraction step is conducted in the pulsed extraction column, the method for separating aromatic compounds contained in naphtha according to the above embodiment of the invention may further include a step of introducing the ionic liquids into the upper end of the pulsed extraction column, and a step of introducing the naphtha into the lower end of the pulsed extraction column.

The configuration of the pulsed extraction column used in the above extraction step may include conventionally known methods and conventionally known elements or device members except for the above-described configuration.

In the method for separating aromatic compounds accroding to the above one embodiment of the invention, conventional methods or devices which are known to be capable of being used for the separation of naphtha except for the above-described configuration can be used without limitation.

### [ADVANTAGEOUS EFFECTS OF THE INVENTION]

According to the present invention, an environmentally friendly and economical separation method that is capable of removing aromatic compounds from naphtha through a simple process with a high efficiency while eliminating the need for multi-stage processes or various processing devices.

In the method for separating aromatic compounds contained in naphtha, components having toxicity or causing corrosion are not generated, thus allowing a more environmentally friendly process to be implemented. Further, even if only the extraction step using the particular ionic liquids is conducted, it is possible to more effectively remove the aromatic compounds from naphtha at high efficiency. Therefore, the need can be eliminated for complicated processes such as a multi-step extraction process or a purification process or installation of various devices, thus allowing a more economical process to be implemented.

### [BRIEF DESCRIPTION OF DRAWING]

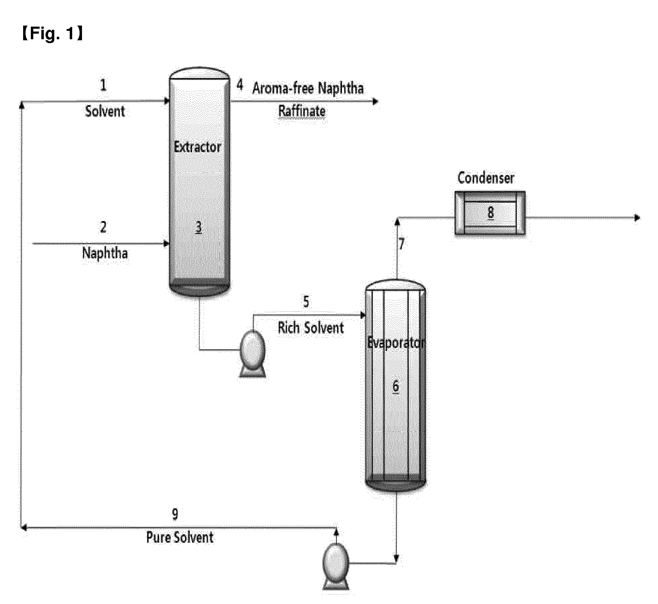

FIG. 1 is a schematic view illustrating the method for separating aromatic compounds from naphtha using the pulsed extraction column.

### [DETAILED DESCRIPTION OF THE EMBODIMENTS]

The present invention will be described in more detail by way of the examples below. However, these examples are intended only to illustrate the present invention, and the contents of the present invention are not intended to be limited by the examples.

### [Preparation Example: Preparation of the ionic liquids]

### 1. Preparation Example 1: Preparation of [2-(1-ethylimidazolium-3-yl-ethoxy)ethyl]-1-ethylimidazolium bis(trifluoromethanesulfonyl)imide

In a 250 ml two-necked flask equipped with a reflux device, 50 g of [2-(1-ethylimidazolium-3-yl-ethoxy)ethyl]-1-ethylimidazolium dibromide [424.17 g/mol] and 78 g of bis(trifluoromethanesulfonyl)imide lithium salt [287.09 g/mol] (2.3 eq.) were placed with water as the solvent and then stirred at room temperature for about 2 h. The reaction product was worked up to remove unreacted materials and then dried in vacuum at 60 °C to give a [2-(1-ethylimidazolium-3-yl-ethoxy)-ethyl]-1-ethylimidazolium bis(trifluoromethane sulfonyl)imide compound with a yield of 98 %.

### 2. Preparation Example 2: Preparation of [4-(1-ethyl piperidinium-3-yl-butoxy)-butyl]-1-ethylpiperidinium bis(trimethanesulfonyl)imide

In a 250 ml two-necked flask equipped with a reflux device, 50 g of [4-(1-ethylpiperidinium-1-yl-butoxy)-butyl]-1-ethylpiperidinium dibromide [514.42 g/mol] and 64 g of bis(trifluoromethane sulfonyl)imide lithium salt [287.09g/mol] (2.3 eq.) were placed with water as the solvent and then stirred at room temperature for about 2 h. The reaction product was worked-up to remove unreacted materials and then dried in vacuum at 60 °C to give a [4-(1-ethylpiperidinium-1-yl-butoxy)-butyl]-1-ethylpiperidinium bis(trifluoromethane sulfonyl)imide compound with a yield of 98 %.

### 3. Preparation Example 2: Preparation of {2-[2-(1-ethylpyrrolidinium-1-yl-ethoxy)-ethoxy]-ethyl}-1-ethylpyrrolidinium bis(tetrafluoroborate)

In a 250 ml two-necked flask equipped with a reflux device, 50 g of {2-[2-(1-ethylpyrrolidinium-1-yl-ethoxy)-ethoxy]-ethyl}-1-ethyl pyrrolidinium dibromide [474.31 g/mol] and 23 g of tetrafluoroborate lithium salt [93.75 g/mol] (2.3 eq.) were placed with water as the solvent and then stirred at room temperature for about 2 h. The reaction product was worked-up to remove unreacted materials and then dried in vacuum at 60 °C to give {2-[2-(1-ethylpyrrolidinium-1-yl-ethoxy)-ethoxy]-ethyl}-1-ethyl-pyrrolidinium bis(tetrafluoroborate) with a yield of 98 %.

### 4. Preparation Example 4: Preparation of (2-{2-(2-(4-ethylmorpholinium-4-yl-ethoxy)-ethoxy]-1-ethyl)-4-ethylmorpholinium(hexafluorophosphate)

In a 250 ml two-necked flask equipped with the reflux device, 50 g of (2-{2-(2-(4-ethylmorpholinium-4-yl-ethoxy)-ethoxy]-ethyl)-4-ethylmorpholinium dibromide [550.37 g/mol] and 32 g of hexafluorophosphate lithium salt [151.91 g/mol] (2.3 eq.) were placed with water as the solvent and then stirred at room temperature for about 2 h. The reaction product was worked-up to remove unreacted materials and then dried in vacuum at 60 °C to give a (2-{2-(2-(4-ethylmorpholinium-4-yl-ethoxy)-ethoxy]-1-ethyl)-4-ethylmorpholinium(hexafluorophosphate) compound with a yield of 98 %.

### [Examples and Comparative Examples: Separation of aromatic compounds from naphtha]

### [Example 1]

5 g of a naphtha sample and 10 g of [2-(1-ethyl imidazolium-3-yl-ethoxy)-ethyl]-1-ethylimidazolium **bis(trifluoromethanesulfonyl)imide** were mixed, stirred at 25 °C for 1 h, and then stabilized for 30 min to separate an upper-layer liquid and a lower-layer liquid.

Then, the separated upper-layer liquid was analyzed using gas chromatography (Young Lin equipment, model #: YL6100), equipped with FID and FFAP columns. In the lower-layer liquid, the extraction rate of the main aromatic compounds was analyzed using Bruker Nuclear Magnetic Resonance (400 MHz/52 MM, Coil No: 944067A).

After extraction, the removal rate of the main aromatic compounds contained in [2-(1-ethylimidazolium-3-yl-ethoxy)-ethyl]-1-ethylimidazolium bis(trifluoromethanesulfonyl) was as shown in Table 1 below.

### [Examples 2 to 20]

The aromatic compounds in naphtha were extracted by conducting a process in the same manner as in Example 1, except that the kinds of the bis-imidazolium-based ionic liquid including an ether group and the bis-biimidazolium-based ionic liquid including an ether group, represented by the above Formulae 1 to 4, were changed and used as shown in Table 1 below.

After extraction, the extraction removal rates of the main aromatic compounds contained in the ionic liquid extraction solvent were as shown in Table 1 below.

### [Comparative Example]

5 g of naphtha sample and 10 g of sulfolane were mixed, stirred at 25 °C for 1 h, and then stabilized for 30 min to separate an upper-layer liquid and a lower-layer liquid.

Then, the separated upper-layer liquid was analyzed using gas chromatography (Young Lin equipment, model #: YL6100), equipped with FID and FFAP columns. In the lower-layer liquid, the extraction rate of the main aromatic compounds was analyzed using Bruker Nuclear Magnetic Resonance (400 MHz/52 MM, Coil No: 944067A).

The removal rates of the main aromatic compounds contained in the main aromatic compounds were shown in Table 1 below.

**[Table 1]**

| Class | Kinds of ionic liquids (Formulae 1 to 3) | Extraction rate (%) | | | |
|---|---|---|---|---|---|
| | | Benzene | Toluene | Mixed xylene | Ethyl benzene |
| Ex.1 | 2-(1-ethylimidazolium-3-yl-ethoxy)ethyl]-1-ethylimidazolium bis(trifluoromethane sulfonyl)imide | 97 | 97 | 95 | 96 |
| Ex. 2 | [2-(1-butylimidazolium-3-yl-ethoxy)-ethyl]-1-butylimidazolium bis(trifluoromethane sulfonyl)imide | 95 | 95 | 92 | 93 |
| Ex. 3 | [4-(1-ethylimidazolium-3-yl-butoxy)-butyl]-1-ethyl imidazolium bis(trifluoro methane sulfonyl)imide | 96 | 95 | 94 | 95 |
| Ex. 4 | {2-[2-(1-ethylimidazolium-3-yl-ethoxy)-ethoxy]-ethyl}-1-ethylimidazolium bis(trifluoromethane sulfonyl)imide | 99 | 98 | 96 | 97 |
| Ex. 5 | (2-{2-[2-(1-ethylimidazolium-3-yl-ethoxy)-ethoxy]-ethoxy]-ethyl)-1-ethylimidazolium bis(trifluoromethane sulfonyl)imide | 99 | 98 | 98 | 98 |
| Ex. 6 | [2-(1-ethylpiperidinium-1-yl-ethoxy)ethyl]-1-ethylpiperidinium bis(trifluoromethanesulfonyl)imide | 96 | 95 | 93 | 94 |
| Ex. 7 | [2-(1-butylpiperidinium-1-yl-ethoxy)ethyl]-1-butyl piperidinium bis(tri fluoromethanesulfonyl) imide | 94 | 93 | 91 | 92 |
| Ex. 8 | 4-(1-ethylpiperidinium-1-yl-butoxy)-butyl]-1-ethyl piperidinium bis(tri fluoromethanesulfonyl) imide | 95 | 94 | 93 | 94 |
| Ex. 9 | {2-[2-(1-ethylpiperidinium -1-yl-ethoxy)-ethoxy]-ethyl}-1-ethylpiperidinium bis(trifluoromethane sulfonyl)imide | 99 | 98 | 96 | 96 |
| Ex. 10 | (2-{2-[2-(1-ethylpiperidinium-1-yl-ethoxy)-ethoxy]-ethoxy}-ethyl)-1-ethylpiperidinium bis(trifluoromethanesulfonyl)imide | 99 | 98 | 97 | 98 |
| Ex. 11 | [2-(1-ethylpyrrolidinium-1-yl-ethoxy)ethyl]-1-ethylpyrrolidinium-bis(trifluoromethane sulfonyl)imide | 96 | 95 | 93 | 93 |
| Ex. 12 | [2-(1-butylpyrrolidinium-1-yl-ethoxy)-ethyl]-1-butylpyrrolidinium-bis(trifluoromethane sulfonyl)imide | 94 | 92 | 91 | 92 |
| Ex. 13 | [4-(1-ethylpyrrolidinium-1-yl-butoxy)-butyl]-1-ethylpyrrolidinium bis(trifluoromethane sulfonyl)imide | 95 | 94 | 92 | 93 |
| Ex. 14 | {2-[2-(1-ethylpyrrolidinium-1-yl-ethoxy)-ethoxy]-ethyl]-1-ethylpyrrolidinium bis(trifluoromethane sulfonyl)imide | 98 | 97 | 95 | 96 |
| Ex. 15 | (2-{2-[2-(1-ethylpyrrolidinium-1-yl-ethoxy)-ethoxy]-ethoxy}-ethyl)-1-ethylpyrrolidinium bis(trifluoromethane sulfonyl)imide | 99 | 98 | 97 | 97 |
| Ex. 16 | [2-(4-ethylmorpholinium 4-yl-ethoxy)-ethyl]-4-ethylmorpholinium bis(trifluoromethane sulfonyl)imide | 98 | 98 | 96 | 97 |
| Ex. 17 | [2-(4-butylmorpholinium-4-yl-ethoxy)-ethyl]-4-butylmorpholinium bis(trifluoromethane sulfonyl)imide | 96 | 96 | 94 | 95 |
| Ex. 18 | [4-(4-ethylmorpholinium-4-yl-butoxy)-butyl]-4-ethyl-morpholinium bis(trifluoromethanesulfonyl) imide | 97 | 97 | 96 | 96 |
| Ex. 19 | {2-[2-(4-ethylmorpholinium 4-yl-ethoxy)-ethoxy]-ethyl}-4-ethylmorpholinium bis(trifluoromethane sulfonyl)imide | 99 | 98 | 96 | 97 |
| Ex. 20 | (2-{2-[2-(4-ethylmorpholinium-4-yl-ethoxy)-ethoxy]-ethoxy]-ethyl)-4-ethylmorpholinium bis(trifluoromethanesulfonyl)imide | 99 | 99 | 97 | 98 |
| Com. Ex. | Sulfolane | 28 | 14 | 12 | 15 |

As shown in Table 1 above, it was confirmed that, in the case of using the ionic liquids in Examples 1 to 20 as an extraction solvent, the interaction with the aromatic compounds was active, so relatively, electronegativity can more effectively extract the aromatic compounds from naphtha.

### [Examples 20 to 26]

The aromatic compounds were extracted from naphtha by conducting a process in the same manner as Example 1, except that two kinds of ionic liquids different from each other were used instead of using only one kind of the ionic liquid as the extraction solvent.

After extraction, the extraction removal rates of the main aromatic compounds contained in the ionic liquid-based extraction solvent were as shown in Table 2.

**[Table 2]**

| Class | Kind of ionic liquids (Formula 1)* | | Extraction rate (%) | | | |
|---|---|---|---|---|---|---|
| | A | B | Benzene | Toluene | Mixed xylene | Ethyl benzene |
| Ex. 1 | [2-(1-ethylimidazolium-3-yl-ethoxy)-ethyl]-1-ethyl-imidazolium bis(trifluoromethanesulfonyl)imide | | 97 | 97 | 95 | 96 |
| Ex. 20 | [2-(1-ethyl imidazolium-3-yl-ethoxy)-ethyl]-1-ethylimidazolium bis(trifluoromethanesulfon yl)imide | [2-(1-ethyl piperidinium-1-yl-ethoxy) ethyl]-1-ethyl piperidinium bis(trifluoro methanesulfonyl)imide | 97 | 97 | 96 | 96 |
| Ex. 21 | [2-(1-butylimidazolium-3-yl-ethoxy)-ethyl] -1-butyl imidazolium bis(trifluoromethanesulfon yl)imide | [4-(1-ethylpyridinium-1-yl-butoxy) -butyl]-1-ethylpyrrolidinium bis(trifluoromethanesulfo nyl) imide | 96 | 95 | 94 | 94 |
| Ex. 22 | {2-[2-(1-ethylimidazolium-3-yl-ethoxy)-ethoxy]-ethyl} -1-ethyl-imidazolium bis(trifluoromethanesulfon yl)imide | [2-(4-ethylmorpholinium-4-yl-ethoxy)-ethyl]-4-ethylmorpholinium bis(trifluoromethanesulfo nyl) imide | 98 | 97 | 96 | 97 |
| Ex. 23 | [2-(1-butyl-piperidinium-1-yl-ethoxy) ethyl]-1-butyl piperidinium bis(trifluoromethanesulfon yl)imide | [4-(4-ethylmorpholinium-4-yl-butoxy)-butyl]-4-ethylmorpholinium bis(trifluoromethanesulfo nyl)imide | 97 | 97 | 95 | 96 |
| Ex. 24 | {2-[2-(1-ethylpyrrolidinium-1-yl-ethoxy)-ethoxy]-ethyl} -1-ethylpyrrolidinium-bis(trifluoromethanesulfon yl) imide | [2-(4-butylmorpholinium-4-yl-ethoxy)ethyl] -4-butylmorpholinium bis(trifluoromethane sulfonyl) imide | 97 | 96 | 95 | 95 |
| Ex. 25 | [2-(1-ethylpyrrolidinium-1-yl-ethoxy)-ethyl]-1-ethylpyrrolidiniun-bis(trifluoromethanesulfon yl) imide | [4-(1-ethylpiperidinium-1-yl-butoxy)-butyl]-1-ethylpiperidinium bis(trifluoromethanesulfo nyl)imide | 96 | 95 | 93 | 4 |
| Ex. 26 | (2-{2-[2-(1-ethylpyperidinium-1-yl-ethoxy)-ethoxy]-ethoxy}-ethyl)-1-ethylpiperidinium bis(trifluoromethanesulfon yl)imide | (2-{2-[2-(1-ethyimidazolium-3-yl-ethoxy)-ethoxy]-ethoxy}-ethyl)-1-ethylimidazolium bis(trifluoromethanesulfo nyl)imide | 99 | 99 | 98 | 98 |
| * Used ratio of the ionic liquids (A: B) = 1: 1 | | | | | | |

As shown in Table 2 above, it was confirmed that in the case of using two kinds of the ionic liquids, the extraction effects were exhibited at a level equivalent to or superior to that of Example 1 using one kind of the ionic liquid.

### [Examples 27 to 33]

The aromatic compounds in naphtha were extracted by conducting a process in the same manner as in Example 1, except that [2-(1-ethylpyrrolidinium-1-yl-ethoxy)-ethyl]-1-ethylpyrrolidinium bis(trifluoromethanesulfonyl)imide was used as the extraction solvent, and the used amounts of the naphtha samples were changed as shown in Table 3 below.

After the extraction, the extraction removal rates of the main aromatic compounds contained in the ionic liquid-based extraction solvent were as shown in Table 3 below.

**[Table 3]**

| Class | Naphtha sample (g)* | Extraction rate (%) | | | |
|---|---|---|---|---|---|
| | | Benzene | Toluene | Mixed xylene | Ethyl benzene |
| Example 27 | 0.5 | 100 | 100 | 100 | 100 |
| Example 28 | 1 | 100 | 100 | 100 | 100 |
| Example 11 | 5 | 96 | 95 | 93 | 93 |
| Example 29 | 10 | 89 | 87 | 85 | 86 |
| Example 30 | 20 | 82 | 78 | 75 | 76 |
| Example 31 | 30 | 72 | 66 | 63 | 65 |
| Example 32 | 40 | 61 | 54 | 50 | 52 |
| Example 33 | 50 | 50 | 47 | 44 | 45 |
| * Amount of naphtha sample applied based on 10 g of the inoic liquid extraction solvent | | | | | |

### [Examples 34 to 36]

The aromatic compounds in naphtha were extracted by conducting a process in the same manner as in Example 1, except that [2-(1-ethylpyrrolidinium-1-yl-ethoxy)-ethyl]-1-ethylpyrrolidinium bis(trifluoromethanesulfonyl)imide was used as the extraction solvent, the throughput of naphtha applied was changed to 5 times by weight with respect to the bis-biimidazolium-based ionic liquid including an ether group, and the extraction was conducted by changing the extraction steps.

After the extraction, the extraction removal rates of the main aromatic compounds contained in naphtha were as shown in Table 4 below.

**[Table 4]**

| Class | Extraction step* | Extraction rate (%) | | | |
|---|---|---|---|---|---|
| | | Benzene | Toluene | Mixed xylene | Ethyl benzene |
| Example 33 | 1 | 50 | 47 | 44 | 45 |
| Example 34 | 2 | 71 | 69 | 67 | 68 |
| Example 35 | 3 | 91 | 89 | 88 | 88 |
| Example 36 | 4 | 100 | 100 | 100 | 100 |

As shown in Table 4 above, it was confirmed that if the amount of naphtha was inceased compared to the ionic liquids, the extraction efficiency was secured up to 100 % by repeating the extraction steps of contacting the ionic liquid and the naphtha twice to four times.

### [Examples 37 to 44]

The aromatic compounds in naphtha were extracted by conducting a proces in the same manner as in Example 1, except that [2-(4-ethylmorpholinium-4-yl-ethoxy)ethyl]-4-ethyl-morpholinium bis(trifluoromethane sulfonyl)imide was used as the extraction solvent, and the extraction temperature was changed as shown in Table 5 below.

After extraction, the extraction removal rates of the main aromatic compounds contained in naphtha were as shown in Table 5 below.

**[Table 5]**

| Class | Extraction temperature (°C) | Extraction rate (%) | | | |
|---|---|---|---|---|---|
| | | Benzene | Toluene | Mixed xylene | Ethyl benzene |
| xample 16 | 25 | 98 | 98 | 96 | 97 |
| Example 37 | 30 | 96 | 96 | 95 | 95 |
| Example 38 | 40 | 94 | 94 | 92 | 93 |
| Example 39 | 50 | 91 | 90 | 88 | 89 |
| Example 40 | 60 | 89 | 88 | 86 | 87 |
| Example 41 | 70 | 87 | 86 | 84 | 85 |
| Example 42 | 80 | 85 | 84 | 82 | 83 |
| Example 43 | 90 | 82 | 81 | 80 | 80 |
| Example 44 | 100 | 80 | 79 | 76 | 77 |

As shown in Table 5 above, it could be confirmed that, even if the extraction temperature was changed from 25 °C to 100 °C, in Examples 37 to 44, the extraction rates of the aromatic compounds in naphtha were nearly 80 %, resulting in excellent extraction efficiency.

### [Examples 45-51]

The aromatic compounds in naphtha were extracted by conducting a process in the same condition and manner as in Example 1, except that {2-[2-(1-ethylpiperidinium-1-yl-ethoxy)-ethoxy]-ethyl}-1-ethyl-piperidinium bis(trifluoromethanesulfonyl)imide was used as the extraction solvent. After the extraction, hydrocarbons of the main aromatic compounds contained in the ionic liquid extraction solvent were deaerated under reduced pressure.

At this time, the deaeration rate according to the deaeration conditions of the temperature, pressure, and time were as shown in Table 6 below.

**[Table 6]**

| Class | Deaeration condition of hydrocarbons | | | Deaeration rate (%) | | | |
|---|---|---|---|---|---|---|---|
| | Temperature (°C) | Pressure (mmHg) | Time (h) | enzene | Toluene | Mixed xylene | Ethyl benzene |
| Example 45 | 20 | 70 | 2 | 88 | 90 | 93 | 92 |
| Example 46 | 50 | 20 | 1 | 100 | 100 | 100 | 100 |
| Example 47 | 70 | 70 | 2 | 100 | 100 | 100 | 100 |
| Example 48 | 80 | 150 | 1 | 83 | 85 | 89 | 87 |
| Example 49 | 100 | 50 | 1 | 100 | 100 | 100 | 100 |
| Example 50 | 120 | 100 | 1 | 90 | 94 | 97 | 96 |
| Example 51 | 150 | 30 | 0.5 | 100 | 100 | 100 | 100 |

As shown in Table 6 above, it could be confirmed that ether group-containing bis-imidazolium-based ionic extraction sovlents and bis-biimidazolium-based ionic liquids could be reused in the extraction process without loss under the deaeration condition.

### Examples 52 to 56 and Comparative Example 2

In the separation of the aromatic compounds in naphtha mixture using the pulsed extraction column, the extraction solvent was introduced in the upper end of the pulsed extraction column 3 via the extraction solvent inlet passage 1 at a velocity of 5 mL per min under a room temperature condition and at the same time naphtha was introduced in the lower end of the pulsed extraction column 3 via the naphtha inlet passage 2 at a velocity of 1 mL per min, and thereby the liquid-liquid extraction separation was conducted at a pulsation velocity of 50 m/s for 3 h within the pulsed extraction column.

After the liquid-liquid extraction separation, the raffinate of the upper-layer (naphtha mixture in which the aromatics have been removed) was discharged to the passage 4 of the upper end of the pulsed extraction column. The extraction rate of the aromatic compounds was analyzed using Gas Chromatography (Young Lin equipment, model #: YL6100), equipped with FID and FFAP columns.

After the extraction, the extraction rate (extraction removal rate) of the aromatic compounds contained in each extraction solvent was as shown in Table 11 below.

**[Table 11]**

| Class | Ionic liquid [Extraction solvent] | Extration rate (%) | | | |
|---|---|---|---|---|---|
| | | Benzene | Toluene | Mixed xylene | Ethyl Benzene |
| Comp. Ex. 2 | Sulfolane | 28 | 14 | 12 | 15 |
| Ex. 52 | [2-(1-ethylimidazolium-3-yl-ethoxy)ethyl]-1-ethylimidazolium bis(trifluoromethane sulfonyl)imide | 96 | 95 | 93 | 95 |
| Ex. 53 | {2-[2-(1-ethyl imidazolium-3-yl-ethoxy) - ethoxy]ethylimidazolium bis(trifluoromethane sulfonyl)imide | 99 | 98 | 97 | 97 |
| Ex. 54 | [2-(1-butylpiperidinium-1-yl-ethoxy)-ethyl]-1-butyl piperidinium bis(trifluoromethanesulfonyl)imide | 97 | 96 | 95 | 96 |
| Ex. 55 | [4-(4-ethylmorpholinium 4-yl-butoxy)-butyl]-4-ethylmorpholinium bis(trifluoromethane sulfonyl)imide | 98 | 97 | 96 | 96 |
| Ex. 56 | (2-{2-[2-(1-ehtylpyrrolidinium-1-yl-ethoxy)-ethoxy]-ethoxy}-ethyl)-1-ethylpyrrolidinium bis(trifluoromethane sulfonyl)imide | 99 | 98 | 96 | 97 |

As shown in Table 11 above, in the case of Examples 52 to 56 using a specific ionic liquid having high electronegativity, it appears that an interaction between the ionic liquid and the aromatic compounds contained in naphtha actively occurred, and thereby it was confirmed that high extraction efficiency was obtained.

In contrast, it could be confirmed that Comparative Example 2 using sulfolane had a low extraction effect as compared with the examples.

### Example 57 to 64

The aromatic compounds were extracted from naphtha mixture by conducting a process in the same manner as in Example 52, except that {2-[2-(1-ethylpiperidinium-1-yl-ethoxy)-ethyl]-1-ethylpiperidinium bis(trifluoromethanesulfonyl)imide was used as the extraction solvent and the used amount (introduced amount) of the naphtha mixture was changed as shown in Table 12 below.

After the extraction, the extraction rate (extraction removal rate) of the aromatic compounds was as shown in Table 12 below.

**[Table 12]**

| Class | Naphtha mixture (mL/min)* | Extraction rate (%) | | | |
|---|---|---|---|---|---|
| | | Benzene | Toluene | Mixed xylene | Ethyl benzene |
| Example 57 | 0.25 | 100 | 100 | 100 | 100 |
| Example 58 | 0.5 | 100 | 100 | 100 | 100 |
| Example 59 | 1 | 97 | 96 | 94 | 95 |
| Example 60 | 5 | 88 | 85 | 83 | 84 |
| Example 61 | 10 | 80 | 76 | 72 | 75 |
| Example 62 | 15 | 70 | 64 | 61 | 63 |
| Example 63 | 20 | 59 | 51 | 48 | 50 |
| Example 64 | 25 | 49 | 45 | 42 | 43 |
| * Amount of naphtha mixture applied based on 5 mL/min of the extraction solvent includng the ionic liquids | | | | | |

As shown in Table 12 above, it was confirmed that Examples 57 to 64 could, even in the case of contacting an excess amount of the naphtha mixture compared to the ionic liquids (extraction solvent), secure higher extraction efficiency as compared to Comparative Example 1.

In particular, as has been confirmed from the results of Examples 6 to 9, when using the naphtha in an amount of 0.05 % to 5 % by volume or 0.1 % to 3 % by volume compared to the ionic liquids, a higher extraction efficiency was secured.

### Examples 65 to 67

The aromatic compounds in naphtha were extracted by conducting a process in the same manner as in Example 52, except that {2-[2-(1-ethylpiperidinium-1-yl-ethoxy)-ethyl]-1-ethylpiperidinium bis(trifluoromethanesulfonyl)imide was used as the extraction solvent, the used amount (introduced amount) of naphtha compared to the bis-biimidazolium-based ionic liquid including an ether group was changed to 5 % by volume, and the extraction was conducted by changing the number of times for conducting the separation with the liquid-liquid extraction method.

After the extraction, the extraction rate (extraction removal rate) of the aromatic compounds was as shown in Table 13 below.

**[Table 13]**

| Class | Number of times for conducting the separation steps | Extraction rate (%) | | | |
|---|---|---|---|---|---|
| | | Benzene | Toluene | Mixed xylene | Ethyl benzene |
| Example 65 | 1 | 49 | 45 | 42 | 43 |
| Example 66 | 2 | 70 | 66 | 63 | 64 |
| Example 67 | 3 | 90 | 87 | 86 | 86 |
| Example 68 | 4 | 100 | 100 | 100 | 100 |

As shown in Table 13 above, it was confirmed that, when increasing the amount of naphtha compared to the ionic liquids, the extraction effect was secured up to 100 % as the extraction steps of contacting the ionic liquid and the naphtha were repeated two times to four times.

### Examples 69 to 73

The aromatic compounds in naphtha were extracted by conducting a process in the same manner as in Example 52, except that [4-(4-ethylmorpholium-4-yl-butoxy)-butyl]-4-ethylmorpholium bis(trifluoromethanesulfonyl)imide was used as the extraction solvent, and the velocity of the pulsation generation motor in the pulsed extraction column was changed as shown in Table 14 below.

After the extraction, the extraction rate (extraction removal rate) of the aromatic compounds was as shown in Table 14 below.

**[Table 14]**

| Class | Velocity (m/s) | Extraction rate (%) | | | |
|---|---|---|---|---|---|
| | | Benzene | Toluene | Mixed xylene | Ethyl benzene |
| Example 69 | 10 | 89 | 88 | 87 | 88 |
| Example 70 | 20 | 91 | 90 | 89 | 89 |
| Example 71 | 30 | 93 | 92 | 90 | 91 |
| Example 72 | 40 | 95 | 95 | 93 | 93 |
| Example 55 | 50 | 98 | 97 | 96 | 96 |
| Example 73 | 60 | 99 | 98 | 96 | 97 |

As shown in Table 14 above, when using the ionic liquids as the extraction solvent, even if the pulsation injection velocity within the pulsed extraction column was changed from 10 m/s to 60 m/s, the extraction rate of the aromatic compounds in naphtha was 87 % or more, which confirmed that the extraction efficiency was excellent.

### Examples 74 to 81

The aromatic compounds in naphtha were extracted by conducting a process in the same manner as in Example 52, except that (2-{2-[2-(1-ethylpyrrolidinium-1-yl-ethoxy)-ethoxy]-ethoxy}-ehtyl)-1-ethylpyrrolidinium bis(trifluoromethanesulfonyl)imide was used as the extraction solvent, and the extraction temperature was changed as shown in Table 15 below.

After the extraction, the extraction rate (extraction removal rate) of the aromatic compounds was as shown in Table 15 below.

**[Table 15]**

| Class | Extraction temperature (°C) | Extraction rate (%) | | | |
|---|---|---|---|---|---|
| | | Benzene | Toluene | Mixed xylene | Ethyl benzene |
| Example 56 | 25 | 99 | 98 | 96 | 97 |
| Example 74 | 30 | 97 | 96 | 95 | 95 |
| Example 75 | 40 | 95 | 94 | 92 | 93 |
| Example 76 | 50 | 92 | 91 | 89 | 89 |
| Example 77 | 60 | 90 | 89 | 86 | 87 |
| Example 78 | 70 | 88 | 87 | 84 | 86 |
| Example 79 | 80 | 85 | 84 | 82 | 84 |
| Example 80 | 90 | 83 | 81 | 80 | 80 |
| Example 81 | 100 | 81 | 79 | 76 | 77 |

As shown in Table 15 above, even if the extraction temperature was changed from 25 °C to 100 °C, the extraction rate of the aromatic compounds in naphtha was almost 80 % or more, which confirmed that extraction efficiency was excellent.

### Examples 82 to 88

The aromatic compounds in naphtha were removed in the same conditions and method as in Example 52, except that [2-(1-butylimidazolium-3-yl-ethoxy)-ethyl]-1-butylimidazolium bis(trifluoromethane sulfonyl)imide was used as the extraction solvent. After the separation with a liquid-liquid extraction method, the extraction solvent including the aromatic compounds was discharged to a passage 5 of the lower end of the pulsed extraction column and introduced into the evaporator 6 and deaerated in vacuum under a specific condition.

At this time, the deaeration rate according to the deaeration conditions such as the temperature, pressure, and time was as shown in Table 16 below.

**[Table 16]**

| Class | Deaeration condition of hydrocarbon | | | Deaeration rate (%) | | | |
|---|---|---|---|---|---|---|---|
| | Temperature (°C) | Pressure (mmHg) | Time (h) | enzene | Toluene | Mixed Xylene | thyl benzene |
| xample 82 | 20 | 70 | 2 | 90 | 94 | 96 | 95 |
| Example 83 | 50 | 20 | 1 | 100 | 100 | 100 | 100 |
| Example 84 | 70 | 70 | 2 | 100 | 100 | 100 | 100 |
| Example 85 | 80 | 150 | 1 | 85 | 87 | 89 | 88 |
| Example 86 | 100 | 50 | 1 | 100 | 100 | 100 | 100 |
| Example 87 | 120 | 100 | 1 | 93 | 96 | 98 | 97 |
| Example 88 | 150 | 30 | 0.5 | 100 | 100 | 100 | 100 |

As shown in Table 16 above, it could be confirmed that the extraction solvent used in the removal process of aromatic compounds was deaerated by diversifying the conditions such as temperature (20 to 150 °C), pressure (1 to 200 mmHg), and time (10 min to 120 min), thus easily removing the aromatic compounds.

### Examples 89 to 92 and Comparative Example 3

The deaerated aromatic compounds were discharged to a passage 7 of the upper end of the evaporator and introduced into a condenser 8. The extraction solvent in which aromatic compounds were removed was discharged to a passage 9 of the lower end of the evaporator and again introduced into the extraction solvent inlet passage 1 and reused.

At this time, the loss rate of the extraction solvent according to the deaeration conditions was as shown in Table 17 below.

**[Table 17]**

| Class | Extraction solvent | Loss rate (%) of extraction solvent according to a certain deaeration condition * |
|---|---|---|
| Comparative Example 3 | Sulfolane | 2 ∼ 4 |
| Example 89 | [2-(1-ethylimidazolium-3-yl-ethoxy)ethyl]-1-ethylimidazolium] bis(trifluoromethanesulfonyl) imide | No loss |
| Example 90 | [4-(1-ethylpiperidinium-1-yl-butoxy)-butyl]-1-ethylpiperidinium bis(trifluoromethanesulfonyl) imide | No loss |
| Example 91 | 2-[2-(1-ethylpyridinium-1-yl-ethoxy)-ethoxy]-ethyl}-1-ethylpyrrolidinium bis(tetrafluoroborate) | No loss |
| Example 92 | (2-{2-[2-(4-ethylmorpholium-4-yl-ethoxy)-ethoxy]-ethoxy}-ethyl)-4-ethylmorpholium bis(hexafluorophosphate) | No loss |
| * Deaeration condition: 70 to 80 °C, 50 to 70 torr, 120 min | | |

As shown in Table 17 above, it could be confirmed that the ether group-containing bis-imidazolium-based ionic liquid extraction solvents and bis-biimidazolium-based ionic liquids could be re-used in the extraction process without loss under certain deaeration conditions.

### Examples 101-105 and Comparative Example 11

In the separation of the aromatic compounds in naphtha mixture using the pulsed extraction column, triethylene gylcol or ionic liquids were introduced in the upper end of the pulsed extraction column 3 via the extraction solvent inlet passage 1 at a velocity of 5 mL/min under a room temperature condition, and at the same time naphtha was introduced in the lower end of the pulsed extraction column 3 via the naphtha inlet passage 2 at a velocity of 1 mL/min. Thereby, the liquid-liquid extraction separation was conducted at the pulsation velocity of 50 m/s for 3 h within the pulsed extraction column.

After the liquid-liquid extraction separation, the raffinate of upper-layer (naphtha mixture in which the aromatics had been removed) was discharged to the passage 4 of the upper end of the pulsed extraction column. The extraction rate of the aromatic compounds was analyzed using gas chromatography (Young Lin equipment, model #: YL6100), equipped with FID and FFAP columns.

After the extraction, the extraction rate (extraction removal rate) of the main aromatic compounds contained in each extraction solvent was as shown in Table 21 below.

**[Table 21]**

| Class | Extraction solvent | Extration rate (%) | | | |
|---|---|---|---|---|---|
| | | Benzene | Toluene | Mixed xylene | Ethyl Benzene |
| Comp. Ex. 11 | Sulfolane | 28 | 14 | 12 | 15 |
| Ex. 101 | Triethylene glycol | 34 | 24 | 21 | 22 |
| Ex. 102 | 1,1'-(1,2-ethanediyl) bis(3-methylimidazolium)bis(trifluoromethane sulfonyl)imide | 97 | 94 | 92 | 93 |
| Ex. 103 | 1,1'-(1,4-butanediyl-2-ol)bis(1-ethylpyrrolidinium) bis(trifluoromethane sulfonyl)imide | 96 | 93 | 90 | 92 |
| Ex. 104 | 1,1'-(2,3-butenediyl) bis(1-ethylmorpholinium)bis(trifluoromethane sulfonyl)imide | 98 | 95 | 92 | 93 |
| Ex. 105 | 1,1'-(1,4-butanediyl-2,3-dione)bis(1-ethylpiperidinium) bis(trifluoromethane sulfonyl)imide | 98 | 96 | 93 | 94 |

Referring to Table 21, it could be confirmed that, when using the ionic liquids of Examples 102 to 105 having high electronegativity as the extraction solvent, an interaction between the ionic liquids and the aromatic compounds actively occurred, thereby exhibiting excellent extraction effects as compared to sulfolane of Comparative Example 11 and triethylene glycol of Example 101, the extraction solvents having relatively low electronegativity.

Further, it could be confirmed that when using the triethylene glycol as the extraction solvent, it exhibited a higher extraction effect than when using sulfolane as the extraction solvent.

### Example 106, 107 and Comparative Examples 12 to 16

The aromatic compounds were extracted from the naphtha mixture by conducting a process in the same manner as in Example 101, except that 1,1'-(1,2-ethanediyl)bis(3-methylimidazolium)bis(trifluoromethanesulfonyl)imide was used as the extraction solvent and the used amount (introduced amount) of the naphtha mixture was changed as shown in Table 22 below.

After the extraction, the extraction rate (extraction removal rate) of the aromatic compounds was as shown in Table 22 below.

**[Table 22]**

| Class | Naphtha mixture (mL/min)* | Extraction rate (%) | | | |
|---|---|---|---|---|---|
| | | Benzene | Toluene | Mixed xylene | Ethyl benzene |
| Example 106 | 0.25 | 100 | 100 | 100 | 100 |
| Example 107 | 0.5 | 100 | 100 | 97 | 98 |
| Example 102 | 1 | 97 | 94 | 92 | 93 |
| Comparative Example 12 | 5 | 88 | 83 | 81 | 82 |
| Comparative Example 13 | 10 | 80 | 74 | 70 | 72 |
| Comparative Example 14 | 15 | 70 | 62 | 59 | 61 |
| Comparative Example 15 | 20 | 59 | 49 | 46 | 48 |
| Comparative Example 16 | 25 | 49 | 42 | 40 | 41 |
| * Amount of naphtha mixture applied based on 5 mL/min of the extraction solvent includng the ionic liquids | | | | | |

Referring to Table 22 above, it could be confirmed that, upon extraction of the aromatic compounds in naphtha, Examples 102, 106, and 107 in which the used amount (introduced amount) of naphtha was 0.1 to 3 % by volume with respect to 100 % by volume of the extraction solvent exhibited higher extraction efficiency than Comparative Examples 12 to 16 at more than 90 %.

### Examples 108 to 110

The aromatic compounds in naphtha were extracted by a process conducting in the same manner as in Example 101, except that triethylene glycol was used as the extraction solvent and the extraction was conducted by changing the number of times of conducting the separation with the liquid-liquid extraction method.

After the extraction, the extraction rate (extraction removal rate) of the aromatic compounds was as shown in Table 23 below.

**[Table 23]**

| Class | Number of times of conducting the separation step | Extraction rate (%) | | | |
|---|---|---|---|---|---|
| | | Benzene | Toluene | Mixed xylene | Ethyl benzene |
| Example 101 | 1 | 34 | 24 | 21 | 22 |
| Example 108 | 2 | 55 | 48 | 44 | 45 |
| Example 109 | 3 | 75 | 72 | 71 | 70 |
| Example 110 | 4 | 94 | 92 | 90 | 92 |

Referring to Table 23 above, it could be confirmed that the case of using triethylene glycol as the extraction solvent exhibited excellent extraction efficiency of 90% or more as compared to the case of conducting two to four multi-step processes.

### Examples 111 to 115

The aromatic compounds in naphtha were extracted by conducting a process in the same manner as in Example 101, except that 1,1'-(1,4-butanediyl-2-ol)bis(1-ethylpyrrolidinium)bis(trifluoromethanesulfonyl)imide was used as the extraction solvent, and the velocity of the pulsation generating motor in the pulsed extraction column was changed as shown in Table 4.

After the extraction, the extraction rate (extraction removal rate) of the aromatic compounds was as shown in Table 24 below.

**[Table 24]**

| Class | Velocity (m/s) | Extraction rate (%) | | | |
|---|---|---|---|---|---|
| | | Benzene | Toluene | Mixed xylene | Ethyl benzene |
| Example 111 | 10 | 87 | 86 | 85 | 86 |
| Example 112 | 20 | 89 | 88 | 86 | 87 |
| Example 113 | 30 | 91 | 90 | 88 | 89 |
| Example 114 | 40 | 93 | 92 | 90 | 91 |
| Example 103 | 50 | 96 | 93 | 90 | 92 |
| Example 115 | 60 | 98 | 95 | 92 | 93 |

Referring to Table 24 above, when using the ionic liquids as the extraction solvent, even if the pulsation injection velocity within the pulsed extraction column was changed from 10 m/s to 60 m/s, the extraction rate of the aromatic compounds in naphtha was 85 % or more, which confirmed that the extraction efficiency was excellent.

### Examples 116 to 123

The aromatic compounds in naphtha were extracted by conducting a process in the same manner as in Example 101, except that 1,1'-(2,3-butanediyl)bis(1-ethylmorpholium)bis(trifluoromethanesulfonyl)imide was used as the extraction solvent and the extraction temperature was changed as shown in Table 25 below.

After the extraction, the extraction rate (extraction removal rate) of the aromatic compounds was as shown in Table 25 below.

**[Table 25]**

| Class | Extraction temperature (°C) | Extraction rate (%) | | | |
|---|---|---|---|---|---|
| | | Benzene | Toluene | Mixed xylene | Ethyl benzene |
| Example 14 | 25 | 98 | 95 | 92 | 93 |
| Example 116 | 30 | 96 | 93 | 89 | 91 |
| Example 117 | 40 | 94 | 91 | 86 | 88 |
| Example 118 | 50 | 91 | 88 | 83 | 86 |
| Example 119 | 60 | 88 | 86 | 81 | 84 |
| Example 120 | 70 | 86 | 84 | 79 | 81 |
| Example 121 | 80 | 83 | 82 | 77 | 79 |
| Example 122 | 90 | 81 | 80 | 74 | 76 |
| Example 123 | 100 | 78 | 77 | 70 | 72 |

Referring to Table 25 above, even if the extraction temperature was changed from 25 °C to 100 °C, the extraction rate of the aromatic compounds in naphtha was 80 % or more, which confirmed that the extraction efficiency was excellent.

### Examples 124 to 130

The aromatic compounds in naphtha were removed in the same conditions and method as in Example 101, except that 1,1'-(1,4-butanediyl-2,3-dione)bis(1-ethylpiperidinium)bis(trifluoromethane sulfonyl) imide was used as the extraction solvent. After the separation with a liquid-liquid extraction method, the extraction solvent including the aromatic compounds was discharged to the passage 5 of the lower end of the pulsed extraction column, introduced in the evaporator 6 and deaerated in a vaccum under certain conditions.

At this time, the deaeration rate according to the deaeration conditions such as the temperature, pressure, and time were as shown in Table 26 below.

**[Table 26]**

| Class | Deaeration condition of hydrocarbon | | | Deaeration rate (%) | | | |
|---|---|---|---|---|---|---|---|
| | Temperature (°C) | Pressure (mmHg) | Time (h) | enzene | Toluene | Mixed xylene | Ethyl benzene |
| Example 124 | 20 | 70 | 2 | 89 | 93 | 95 | 94 |
| Example 125 | 50 | 20 | 1 | 100 | 100 | 100 | 100 |
| Example 126 | 70 | 70 | 2 | 100 | 100 | 100 | 100 |
| Example 127 | 80 | 150 | 1 | 84 | 86 | 89 | 88 |
| Example 128 | 100 | 50 | 1 | 100 | 100 | 100 | 100 |
| Example 129 | 120 | 100 | 1 | 92 | 95 | 98 | 97 |
| Example 130 | 150 | 30 | 0.5 | 100 | 100 | 100 | 100 |

Referring to Table 26 above, it could be confirmed that the extraction solvent used in the removal process of aromatic compounds could be deaerated by diversifying the conditions such as temperature (20 to 150 °C), pressure (1 to 200 mmHg), and time (10 min to 120 min), thus easily removing the aromatic compounds.

### Examples 131 to 135, and Comparative Example 17

The deaerated aromatic compounds were discharged to the passage 7 of the upper end of the evaporator and introduced into the condenser 8. The extraction solvent in which aromatic compounds had been removed was discharged to the passage 9 of the lower end of the evaporator, and again introduced into the extraction solvent inlet passage 1 and reused.

At this time, the loss rate of the extraction solvent according to the deaeration conditions of Table 27 below was as shown in Table 27 below.

**[Table 27]**

| Class | Extraction solvent | Loss rate of extraction solvent according to a certain deaeration condition (%) |
|---|---|---|
| Comparative Example 17 | Sulfolane | 2∼4 |
| Example 131 | Triethylene glycol | No loss |
| Example 132 | 1,1'-(1,2-ethanediyl)bis(3-methylimidazolium) bis(trifluoro methanesulfonyl)imide | No loss |
| Example 133 | 1,1'-(1,4-butanediyl-2-ol) bis(1-ethylpyrrolidinium) bis(trifluoro methanesulfonyl)imide | No loss |
| Example 134 | 1,1'-(2,3-butenediyl)bis(1-ethylmorpholinylinium) bis(trifluoromethane sulfonyl)imide | No loss |
| Example 135 | 1,1'-(1,4-butanediyl-2,3-dione)bis(1-ethylpiperidinium)bis(trifluoromethane sulfonyl)imide | No loss |
| * Deaeration condition: 70 to 80 °C, 50 to 70 torr, 120 min | | |

Referring to Table 27 above, it could be confirmed that the triethylene glcycol extraction solvent, the bis-imidazolium-based ionic liquid extraction solvent, and the bis-biimidazolium-based ionic liquid extraction solvent which were used in the present process could be reused in the extraction process without loss under certain deaeration conditions.

### [Description of Reference Numerals]

1: Extraction solvent inlet passage
2: Naphtha mixture inlet passage
3: Pulsed extraction column
4: Passage of the upper end of the pulsed extraction column
5: Passage of the lower end of the pulsed extraction column
6: Evaporator
7: Passage of the upper end of the evaporator
8. Condenser
9: Passage of the lower end of the evaporator

## Claims

1. A method for separating aromatic compounds contained in naphtha which comprises the steps of: contacting naphtha with ionic liquids including one or more compounds selected from the group consisting of a triethylene glycol, a bis-imidazolium-based ionic solvent, a bis-piperidinium-based ionic solvent, a bis-pyrrolidinium-based ionic solvent, a bis-morpholinium-based ionic solvent, a bis-imidazolium-based salt including an ether group, a bis-piperidinium-based salt including an ether group, a bis-pyrrolidinium-based salt including an ether group, and a bis-morpholinium-based salt including an ether group; and
separating aromatic compounds from the naphtha contacted with the ionic liquids.

2. The method for separating aromatic compounds contained in the naphtha of claim 1 wherein:
the bis-imidazolium-based salt including an ether group includes a cation in which two imidazolium-based rings are linked via a functional group including one or more ether groups having a total carbon number of 2 to 20, and a halide-based anion having hydrophobicity;
the bis-piperidinium-based salt including an ether group includes a cation in which two piperidinium-based rings are linked via a functional group including one or more ether groups having a total carbon number of 2 to 20, and a halide-based anion having hydrophobicity;
the bis-pyrrolidinium-based salt including an ether group includes a cation in which two pyrrolidinium-based rings are linked via a functional group including one or more ether groups having a total carbon number of 2 to 20, and a halide-based anion having hydrophobicity; and
the bis-morpholinium-based salt including an ether group includes a cation in which two morpholinium-based rings are linked via a functional group including one or more ether groups having a total carbon number of 2 to 20, and a halide-based anion having hydrophobicity.

3. The method for separating aromatic compounds contained in the naphtha of claim 1 wherein
the bis-imidazolium-based salt including an ether group includes a compound represented by Formula 1 below,
the bis-piperidinium-based salt including an ether group includes a compound represented by Formula 2 below,
the bis-pyrrolidinium-based salt including an ether group includes a compound represented by Formula 3 below, and
the bis-morpholium-based salt including an ether group includes a compound represented by Formula 4 below: 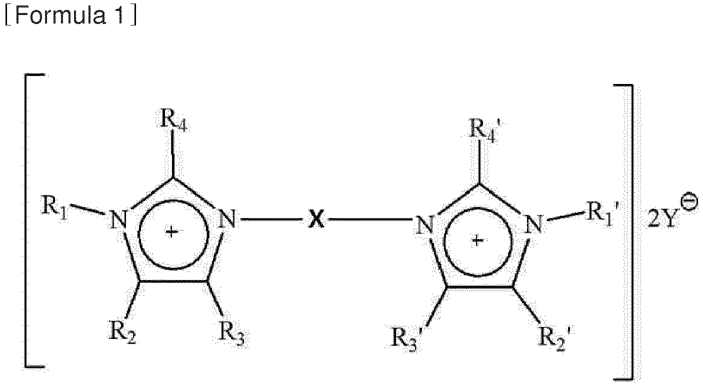
in the above Formula 1, R₁ and R₁' may be the same as or different from each other and are each independently a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms, R₂, R₂', R₃, R₃', R₄, and R₄' may be the same as or different from each other and are each independently hydrogen, a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms,
Y is (CF₃SO₂)₂N⁻, BF₄₋, or PF₆⁻, and
X is the following Formula 1 a or Formula 1b: 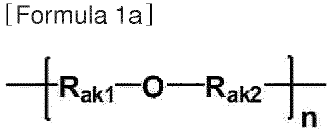 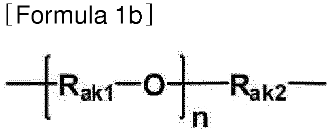
in the Formula 1 a or Formula 1 b, Rₐₖ₁ and Rₐₖ₂ are each independently a linear or branched alkylene group having 1 to 10 carbon atoms, and n is an integer from 1 to 5; 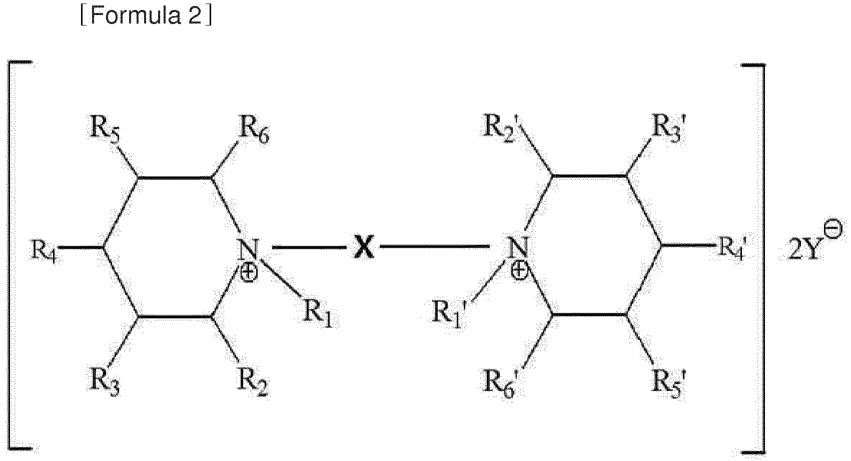
in the above Formula 2,
R₁ and R₁' may be the same as or different from each other and are each independently a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms, R₂, R₂', R₃, R₃', R₄, R₄', R₅, R₅', R₆, and R₆' may be the same as or different from each other and are each independently hydrogen, a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms,
Y is (CF₃SO₂)₂N⁻, BF₄₋, or PF₆⁻, and
X is the following Formula 2a or Formula 2b: 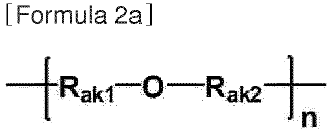 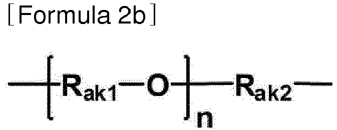 in the above Formula 2a or Formula 2b, Rₐₖ₁ and Rₐₖ₂ are each independently a linear or branched alkylene group having 1 to 10 carbon atoms, and n is an integer from 1 to 5; 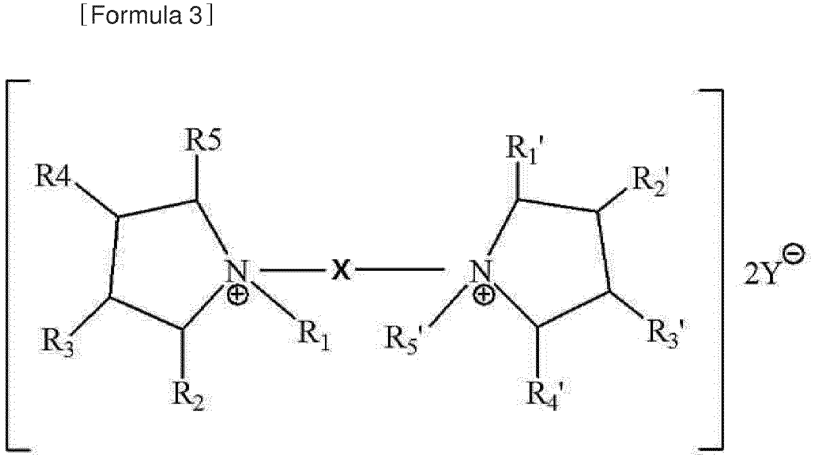
in the above Formula 3,
R₁ and R₁' may be the same as or different from each other and are each independently a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms, R₂, R₂', R₃, R₃', R₄, R₄', R₅, and R₅' may be the same as or different from each other and are each independently hydrogen, a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms,
Y is (CF₃SO₂)₂N⁻, BF₄₋, or PF₆⁻, and
X is the following Formula 3a or Formula 3b: 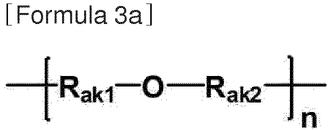 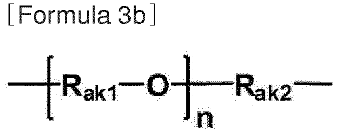 in the above Formula 3a or Formula 3b, Rₐₖ₁ and Rₐₖ₂ are each independently a linear or branched alkylene group having 1 to 10 carbon atoms, and n is an integer from 1 to 5; 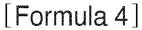 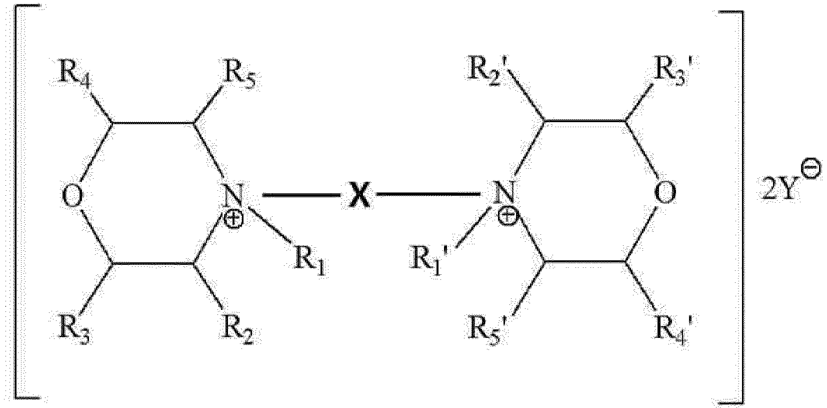
in the above Formula 4,
R₁ and R₁' may be the same as or different from each other and are each independently a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms, R₂, R₂', R₃, R₃', R₄, R₄', R₅, and R₅' may be the same as or different from each other and are each independently hydrogen, a linear or branched alkyl group having 1 to 10 carbon atoms, a linear or branched alkenyl group having 2 to 10 carbon atoms, a linear or branched alkoxy group having 1 to 10 carbon atoms, or a linear or branched alkyl carboxyl group having 1 to 10 carbon atoms,
Y is (CF₃SO₂)₂N⁻, BF₄₋, or PF₆⁻, and
X is the following Formula 4a or Formula 4b: 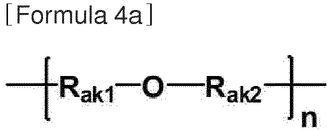 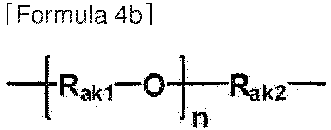 in the above Formula 4a or Formula 4b, Rₐₖ₁ and Rₐₖ₂ are each independently a linear or branched alkylene group having 1 to 10 carbon atoms, and n is an integer from 1 to 5.

4. The method for separating aromatic compounds contained in the naphtha of claim 1,
wherein the bis-imidazolium-based ionic solvent includes one or more compounds selected from the group consisting of 1,1'-(1,2-ethanediyl)bis(3-methylimidazolium) bis(trifluoromethanesulfonyl)imide, 1,1'-(1,4-butanediyl)bis(3-ethylimidazolium) bis(trifluoromethane sulfonyl)imide, 1,1'-(2,4'-butenediyl)bis(3-butylimidazolium) bis(trifluoromethanesulfonyl)imide, 1,1'-(1,4-butanediyl-2-one)bis(3-ethylimidazolium) bis(trifluoromethane sulfonyl)imide, 1,1'-(1,4-butanediyl-2,3-dione)bis(3-butylimidazolium) bis(trifluoromethanesulfonyl)imide, 1,1'-(1,4-butanediyl-2-ol)bis(3-methylimidazolium) bis(trifluoro methanesulfonyl) imide, and 1,1'-(1,4-butanediyl-2,3-diol)bis(3-ethylimidazolium) bis(trifluoromethane sulfonyl)imide, 1,1'-(1,2-ethanediyl)bis(3-methylimidazolium) bis(tetrafluoroborate), 1,1'-(1,4-butanediyl) bis(3-ethylimidazolium) bis(tetrafluoroborate), 1,1'-(2,4-betenediyl)bis(3-butylimidazolium) bis(tetrafluoroborate), 1,1'-(1,4-butanediyl-2-one)bis(3-ethylimidazolium) bis(tetrafluoroborate), 1,1'-(1,4-butanediyl-2,3-dione)bis(3-ethylimidazolium) bis(tetrafluoroborate), 1,1'-(1,4-butanediyl-2-ol) bis(3-ethylimidazolium) bis(tetrafluoroborate), and 1,1'-(1,4-butanediyl-2,3-diol) bis(3-ethylimidazolium) bis(tetrafluoroborate), 1,1'-(1,2-ethanediyl)bis(3-methylimidazolium) bis(hexafluorophosphate), 1,1'-(1,4-butanediyl)bis(3-ethylimidazolium) bis(hexafluorophosphate), 1,1'-(2,4-butenediyl) bis(3-butyl-imidazolium) bis(hexafluorophosphate), 1,1'-(1,4-butanediyl-2-pyridyl) bis(3-ethylimidazolium) bis(hexamethylene fluorophosphate), 1,1'-(1,4-butanediyl-2,3-dione) bis(3-butylimidazolium) bis(hexafluorophosphate), 1,1'-(1,4-butanediyl-2-ol)bis(3-ethylimidazolium) bis(hexafluorophosphate), and 1,1'-(1,4-butanediyl-2,3-diol) bis(3-ethylimidazolium) bis(hexafluorophosphate).

5. The method for separating aromatic compounds contained in the naphtha of claim 1,
wherein the bis-pyrrolidinium-based ionic solvent includes one or more compounds selected from the group consisting of 1,1'-(1,4-butanediyl)bis(1-ethylpyrrolidinium) bis(trifluoromethane sulfonyl)imide, 1,1'-(1,4-butanediyl)bis(1-butylpyrrolidinium)bis(trifluoromethane sulfonyl)imide, 1,1'-(1,4-butanediyl-2-ol)bis(1-ethylpyrrolidinium)bis(trifluoromethane sulfonyl)imide, 1,1'-(1,4-butanediyl-2,3-diol) bis(1-ethylpyrrolidinium) bis(trifluoromethane sulfonyl)imide, 1,1'-(2,3-butendiyl)bis(1-ethylpyrrolidinium)bis(trifluoromethanesulfonyl)imide, 1,1'-(1,4-butanediyl-2,3-dione) bis(1-ethylpyrrolidinium) bis(trifluoromethane sulfonyl)imide, 1,1'-(1,4-butanediyl) bis(1-ethylpyrrolidinium) bis(tetrafluoroborate), 1,1'-(1,4-butanediyl)bis(1-butylpyrrolidinium) bis(tetrafluoroborate), 1,1'-(1,4-butanediyl-2-ol)bis(1-ethylpyrrolidinium)bis(tetrafluoroborate), 1,1'-(1,4-butanediyl-2,3-diol)bis(1-ethylpyrrolidinium) bis(tetrafluoroborate), 1,1'-(2,3-butenediyl)bis(1-ethylpyrrolidinium) bis(tetrafluoroborate), 1,1'-(1,4-butanediyl-2,3-dione)bis(1-ethylpyrrolidinium) bis(tetrafluoroborate), 1,1'-(1,4-butanediyl)bis(1-ethylpyrrolidinium) bis(hexafluorophosphate), 1,1'-(4-butanediyl)bis(1-butylpyrrolidinium) bis(hexafluorophosphate), 1,1'-(1,4-butanediyl-2-ol)bis(1-ethylpyrrolidinium)bis(hexafluorophosphate), 1,1'-(1,4-butanediyl-2,3-diol)bis(1-ethylpyrrolidinium) bis(hexafluorophosphate), 1,1'-(2,3-butenediyl) bis(1-ethylpyrrolidinium) bis(hexafluorophosphate), and 1,1'-(1,4-butanediyl-2,3-dione) bis(1-ethylpyrrolidinium) bis(hexafluorophosphate);
the bis-morpholinium-based ionic solvent includes one or more compounds selected from the group consisting of 1,1'-(1,4-butanediyl)bis(1-ethylmorpholinium)bis(trifluoromethane sulfonyl)imide, 1,1'-(1,4-butanediyl)bis(1-butylmorpholinium) bis(trifluoro methane sulfonyl)imide, 1,1'-(1,4-butanediyl-2-ol)bis(1-ethylmorpholinium)bis(trifluoromethane sulfonyl) imide, 1,1'-(1,4-butanediyl-2,3-diol)bis(1-ethylmorpholinium) bis(trifluoromethane sulfonyl)imide, 1,1'-(2,3-butenediyl)bis(1-ethylmorpholinium)bis(trifluoromethane sulfonyl)imide, 1,1'-(1,4-butanediyl-2,3-dione)bis(1-ethylmorpholinium)bis(trifluoro methane sulfonyl)imide, 1,1'-(1,4-butanediyl) bis(1-ethylmorpholinium)bis(tetrafluoroborate), 1,1'-(1,4-butanediyl)bis(1-butylmorpholinium) bis(tetrafluoroborate), 1,1'-(1,4-butanediyl-2-ol) bis(1-ethylmorpholinium) bis(tetrafluoroborate), 1,1'-(1,4-butanediyl-2,3-diol) bis(1-ethylmorpholinium) bis(tetrafluoroborate), 1,1'-(2,3-butenediyl)bis(1-ethylmorpholinium) bis(tetrafluoroborate), 1,1'-(1,4-butanediyl-2,3-dione) bis(1-ethylmorpholinium)bis(tetrafluoroborate), 1,1'-(1,4-butanediyl) bis(1-ethylmorpholinium)bis(hexafluorophosphate), 1,1'-(1,4-butanediyl) bis(1-butylmorpholinium) bis(hexafluorophosphate), 1,1'-(1,4-butanediyl-2-ol) bis(1-ethyl-morpholinium) bis(hexafluorophosphate), 1,1'-(1,4-butanediyl-2,3-diol) bis(1-ethylmorpholinium) bis(hexafluorophosphate), 1,1'-(2,3-butendiyl)bis(1-ethylmorpholinium) bis(hexafluorophosphate), and 1,1'-(1,4-butanediyl-2,3-dione) bis(1-ethylmorpholinium)bis(hexafluorophosphate); and
the bis-piperidinium-based ionic solvent includes one or more compounds selected from the group consisting of 1,1'-(1,4-butanediyl)bis(1-ethylpiperidinium) bis(trifluoromethane sulfonyl)imide, 1,1'-(1,4-butanediyl) bis(1-butylpiperidinium) bis(trifluoromethane sulfonyl) imide, 1,1'-(1,4-butanediyl-2-ol)bis(1-ethyl-piperidinium) bis(trifluoromethane sulfonyl)imide, 1,1'-(1,4-butanediyl-2,3-diol) bis(1-ethylpiperidinium) bis(trifluoro methane sulfonyl)imide, 1,1'-(2,3-butanediyl)bis(1-ethylpiperidinium) bis(trifluoromethane sulfonyl)imide, 1,1'-(1,4-butanediyl-2,3-dione)bis(1-ethylpiperidinium) bis(trifluoromethane sulfonyl)imide, 1,1'-(1,4-butanediyl)bis(1-ethylpiperidinium)bis(tetrafluoroborate), 1,1'-(1,4-butandiyl)bis(1-butylpiperidinium) bis(tetrafluoroborate), 1,1'-(1,4-butanediyl-2-ol)bis(1-ethylpiperidinium) bis(tetrafluoroborate), 1,1'-(1,4-butanediyl-2,3-diol)bis(1-ethyl-piperidinium) bis(tetrafluoroborate), 1,1'-(2,3-butenediyl)bis(1-ethylpiperidinium) bis(tetrafluoroborate), 1,1'-(1,4-butanediyl-2,3-dione)bis(1-ethylpiperidinium) bis(tetrafluoroborate), 1,1'-(1,4-butanediyl)bis(1-ethylpiperidinium) bis(hexafluorophosphate), 1,1'-(1 ,4-butanediyl)bis(1-butylpiperidinium) bis(hexafluorophosphate), 1,1'-(1,4-butanediyl-2-ol)bis(1-ethylpiperidinium) bis(hexafluorophosphate), 1,1'-(1,4-butanediyl-2,3-diol)bis(1-ethylpiperidinium) bis(hexafluorophosphate), 1,1'-(2,3-butenediyl)bis(1-ethylpiperidinium) bis(hexafluorophosphate), and 1,1'-(1,4-butanediyl-2,3-dione) bis(1-ethylpiperidinium)bis(hexafluorophosphate).

6. The method for separating aromatic compounds contained in the naphtha of claim 1,
wherein the naphtha is used in an amount of 0.05 to 5 % by volume compared to the ionic liquids.

7. The method for separating aromatic compounds contained in the naphtha of claim 1,
wherein the step of contacting the ionic liquid and naphtha is conducted at a temperature of 20 °C. to 100 °C.

8. The method for separating aromatic compounds contained in the naphtha of claim 1,
wherein the step of contacting the ionic liquids and naphtha is conducted for 1 min or more.

9. The method for separating aromatic compounds contained in the naphtha of claim 1,
wherein the step of contacting the ionic liquids and naphtha is conducted for 30 min to 10 h.

10. The method for separating aromatic compounds contained in the naphtha of claim 1,
wherein the step of separating aromatic compounds from naphtha contacted with the ionic liquids is conducted at least once.

11. The method for separating aromatic compounds contained in the naphtha of claim 1,
wherein the step of separating aromatic compounds from naphtha contacted with the ionic liquids includes a step for conducting the separation by a liquid-liquid extraction method.

12. The method for separating aromatic compounds contained in the naphtha of claim 1, which further comprises a step for separating a raffinate obtained in the extraction step.

13. The method for separating aromatic compounds contained in the naphtha of claim 1,
wherein the step of separating aromatic compounds from naphtha contacted with the ionic liquids further comprises a step of conducting deaeration at a temperature of 20 to 150 °C and a pressure of 1 to 200 mmHg.

14. The method for separating aromatic compounds contained in the naphtha of claim 1,
wherein the extraction step is performed in a pulsed extraction column.

15. The method for separating aromatic compounds contained in the naphtha of claim 1,
wherein the pulsation is introduced at a velocity of 10 m/s to 60 m/s in the pulsed extraction column.

16. The method for separating aromatic compounds contained in the naphtha of claim 1, which further comprises the steps of introducing the extraction solvent into the upper end of the pulsed extraction column, and introducing the naphtha into the lower end of the pulsed extraction column.

17. The method for separating aromatic compounds contained in the naphtha of claim 1,
wherein the step of separating aromatic compounds from naphtha contacted with the ionic liquids is a step of conducting the separatiion by a liquid-liquid extraction method.
